# EUROPÄISCHE PATENTSCHRIFT

(11) **EP 4 061 430 B1**
(45) Veröffentlichungstag und Bekanntmachung des Hinweises auf die Patenterteilung: **26.06.2024**
(21) Anmeldenummer: 21815482.1
(22) Anmeldetag: 18.11.2021
(51) Int. Cl.: A61L 2/00, A61B 50/22

(54) **STERILISIERSIEBSCHALE MIT FIXIERGRIFF**
STERILISATION BASKET WITH FIXING HANDLE
PANIER DE STÉRILISATION COMPORTANT UNE POIGNÉE DE FIXATION

(30) Priorität: 24.11.2020 DE 102020131104
(43) Veröffentlichungstag der Anmeldung: 28.09.2022
(73) Patentinhaber: Aesculap AG, 78532 Tuttlingen (DE)
(72) Erfinder: HENKE, Matthias, 78048 Villingen-Schwenningen (DE); LUZ, Sebastian, 78576 Emmingen-Liptingen (DE); KUTSCHER, Ramona, 78187 Geisingen (DE); JAKAB, Mariana, 78532 Tuttlingen (DE)
(74) Vertreter: Winter, Brandl - Partnerschaft mbB
(86) Internationale Anmeldenummer: PCT/EP2021/082180
(87) Internationale Veröffentlichungsnummer: WO 2022/112097

(56) Entgegenhaltungen:
- DE-C1- 10 101 424
- DE-U1- 20 317 905
- US-A- 4 728 504

## Beschreibung

### Technisches Gebiet

Die vorliegende Erfindung betrifft eine Sterilisiersiebschale / einen Sterilisiersiebkorb / eine Sterilisierkorbwanne / eine Siebkorbwanne gemäß dem Oberbegriff des Patentanspruchs 1, insbesondere eine Sterilisiersiebschale, die zum Einsetzen in und Entnehmen aus einer Containerwanne vorgesehen und ausgebildet ist, wofür an wenigstens einer Siebschalenseitenwand wenigstens ein, vorzugsweise bügelförmiger oder henkelförmiger, Siebschalengriff bewegbar, insbesondere schwenkbar, gelagert ist, der einen Greifabschnitt zum manuelle Ergreifen hat, der über einen winklig zu dem Greifabschnitt ausgerichteten Verbindungssteg mit einem Lagerabschnitt verbunden ist, an welchem der Siebschalengriff an einem siebschalenwandseitigen Lager gehalten ist und in zumindest eine (erste) Trageposition / Tragestellung / Ergreifstellung und in eine (zweite) Fixierposition / Fixierstellung / Sicherungsstellung bewegbar, insbesondere schwenkbar, ist. Daneben betrifft die Erfindung ein Sterilisiersiebschalensystem sowie ein Sterilcontainersystem.

### Hintergrund der Erfindung

Im Gesundheitsweisen stellen Medizinprodukte, die direkt mit dem menschlichen Körper in Berührung kommen, ein besonderes Infektionsrisiko für den Patienten dar. Um einen möglichst guten Schutz des Patienten vor Infektionen durch Medizinprodukte zu erreichen, werden hohe Anforderungen an einen hygienischen Status der Medizinprodukte gestellt. Für kritische Medizinprodukte gilt die Forderung nach Sterilität, wobei ein steriles Medizinprodukt frei von vermehrungsfähigen Organismen ist, einschließlich frei von vermehrungsfähigen Sporen. Für eine Wiederverwendung werden die Produkte entsprechend aufbereitet und sterilisiert.

Übliche Verfahren zur Sterilisation sind beispielsweise Hitzesterilisationsverfahren, entweder mittels feuchter Hitze oder mittels Heißluft, Niedertemperatur-Gas-Verfahren, beispielsweise Ethylenoxid-Verfahren, oder Desinfektionsverfahren mit wässrigen Lösungen. Bei diesen Verfahren werden (thermostabile) Medizinprodukte in einem Sterilisiersiebkorb / einer Sterilisiersiebschale / einer Sterilisierkorbwanne / einer Siebkorbwanne zur Sterilisation angeordnet und dem entsprechenden Verfahren unterzogen.

Um zu verhindern, dass insbesondere während eines Waschprozesses, leichte oder kleine Instrumente aus der Sterilisiersiebschale herausgeschleudert werden oder diese durch weitere Umstände ungewollt verlassen, und um zu verhindern, dass während eines Transports in Sterilbarrieresystemen oder auf Transportwägen die Medizinprodukte herausfallen, wird die Sterilisiersiebschale üblicherweise mit einem zugehörigen passenden Siebschalendeckel versehen und bildet zusammen ein Sterilisiersiebschalensystem. Dieser verschließt die Sterilisiersiebschale und verhindert geometriebedingt einen Austritt der Medizinprodukte aus der Sterilisiersiebschale. Normalerweise wird ein Siebschalendeckel bei einem Sterilisiersiebschalensystem mit einer Sterilisiersiebschale lose auf die Sterilisiersiebschale aufgelegt bzw. aufgesetzt. Hiernach werden zwei schwenkbare Siebschalengriffe bzw. Handgriffe der Sterilisiersiebschale, die an zwei gegenüberliegenden Innenseitenflächen der Sterilisiersiebschale angebracht sind, und die bei Aufsetzen des Siebschalendeckels in einer gegenüber der Sterilisiersiebschale oberen/abgewandten Schwenkposition (Ergreif-Position bzw. Trageposition) waren, die das Aufsetzen des Siebschalendeckels ermöglichten, nach unten auf den aufgesetzten Siebschalendeckel geschwenkt und liegen schließlich lose auf dem Siebschalendeckel auf.

### Stand der Technik

Nach dem Stand der Technik dienen Sterilisiersiebschalen bzw.

Sterilisiersiebschalensysteme der Aufnahme medizinischer oder chirurgischer Instrumente über den gesamten Sterilgutkreislauf hinweg, welche während eines Packprozesses auf einer reinen Seite einer Aufbereitungseinheit für Medizinprodukte (AEMP, früher auch zentrale Sterilisiergutversorgungsabteilung genannt) in ein Sterilbarrieresystem gepackt werden, wobei als Sterilbarrieresystem meist Container / Sterilcontainer verwendet werden. Ein solcher Sterilcontainer umfasst eine Containerwanne und einen passenden Containerdeckel, wobei in dem Containerdeckel üblicherweise in einer Öffnung des Containerdeckels ein Filter eingesetzt ist, um gegenüber dem ansonsten hermetisch versiegelten Sterilcontainer eine definierte Verbindung unter Zwischenschaltung des Filters zur äußeren Umgebung bereitzustellen. Um die Sterilisiersiebschale bzw. das Sterilisiersiebschalensystem in einen Sterilcontainer stellen bzw. anordnen zu können, ist es grundsätzlich erforderlich, dass die Sterilisiersiebschale geringere geometrische Außenmaße als eine Innenseite des Sterilcontainers aufweist, da ansonsten die Sterilisiersiebschale auf einem Rand des Sterilcontainers aufsetzen würde. Mit anderen Worten muss also der Sterilcontainer solche geometrischen Innenmaße (Länge, Breite, Höhe) aufweisen, dass die Sterilisiersiebschale bzw. das Sterilisiersiebschalensystem mit entsprechenden geometrischen Außenmaßen vollständig in dem Sterilcontainer aufnehmbar ist. Zwangsläufig ist auch ein Spalt zwischen den Innenseiten des Sterilcontainers und den Außenseiten der Sterilisiersiebschale erforderlich, um ein gutes, schnelles und effektives Einführen der Sterilisiersiebschalen in den Sterilcontainer zu ermöglichen.

Dieser umfängliche Spalt zwischen der Außenseite und der Innenseite führt bei einem Transport des Sterilcontainers jedoch, etwa durch Vibrationen und Beschleunigungen, zu einer Bewegung der Sterilisiersiebschale in dem Sterilcontainer und zu einem entsprechenden Abrieb der aneinander reibenden Flächen an sowohl der Sterilisiersiebschale als auch an dem Sterilcontainer. Darüber hinaus kann eine solche Bewegung zu einem ungewollten Verrutschen des Inhaltes der Sterilisiersiebschalen führen, was insbesondere in einer Beschädigung des Inhaltes resultieren kann. Selbst an dem Siebschalenboden / einer Unterseite der Sterilisiersiebschale angebrachte (Stand-)Füße können ein solches Verrutschen nicht vollständig unterbinden und nutzen sich zudem mit der Zeit ab.

Die DE 101 01 424 C1 offenbart einen Siebkorb mit schwenkbaren Transportgriffen. Diese Griffe können von einer Ruhestellung, in welchem der Griff jeweils im Inneren Volumen des Siebkorbs gelagert ist, in eine vertikale Tragestellung hochgeschwenkt werden.

Die US 4 728 504 A offenbart ein Sterilisiercontainer, in welchen ein Siebkorb einsetzbar ist. Diese Sterilisiercontainer können übereinander gestapelt werden.

Ferner offenbart die DE 203 17 905 U1 eine Siebschale 1 mit einem Tragegriff 9, der längsverschieblich entlang seiner Drehachse gelagert ist und je nach Schwenkzustand mit einem U-förmigen Bügel 15 bzw. einem Vorsprung 14 zusammenwirkt, um gestapelte Siebschalen miteinander zu verriegeln.

### Zusammenfassung der Erfindung

Es ist daher die Aufgabe der Erfindung, die Nachteile aus dem Stand der Technik zu vermeiden oder wenigstens zu mindern und insbesondere eine Sterilisiersiebschale, ein Sterilisiersiebschalensystem, sowie ein Sterilcontainersystem zur Verfügung zu stellen, die bzw. das einen Kratzschutz und einen Transportschutz bereitstellt, einfach und sicher und gut in der Handhabung ist, insbesondere gut für einen Transport und eine Lagerung geeignet ist sowie langlebig und verschleißarm ist. Insbesondere bietet sie bzw. es einem Anwender die Funktion, die Sterilisiersiebschale gut manuell in einen Sterilcontainer einzusetzen und gegenüber dem Sterilcontainer zu fixieren und ein Verrutschen zu verhindern, sowie die Sterilisiersiebschale ebenso einfach, sicher und gut wieder zu entnehmen. Darüber hinaus soll ein Siebschalendeckel einfach und sicher auf die Sterilisiersiebschale aufsetzbar sein, selbst im in dem Sterilcontainer eingesetzten Zustand.

Die Aufgabe der Erfindung wird bei einer gattungsgemäßen Sterilisiersiebschale erfindungsgemäß durch die Merkmale des Anspruchs 1, bei einem gattungsgemäßen Sterilisiersiebschalensystem erfindungsgemäß durch die Merkmale des Anspruchs 8 und bei einem gattungsgemäßen Sterilcontainersystem erfindungsgemäß durch die Merkmale des Anspruchs 9 gelöst.

Der Kern der vorliegenden Erfindung sieht also demzufolge vor, eine solche Sterilisiersiebschale zur Verfügung zu stellen, die kausal bedingt durch die ausgewählte Bewegung des Siebschalengriffs, also insbesondere durch die manuelle Manipulation des Greifabschnitts, in Folge einen speziell vorgesehenen und wirkverbundenen bzw. (wirk)gekoppelten Fixierabschnitt und/oder Andrückabschnitt oder Element, der/das insbesondere dafür angepasst ist, als Kontaktpunkt oder Kontaktfläche in Anlage zu kommen, in Richtung außerhalb der Sterilisiersiebschale zu bewegen und/oder zu deformieren. Durch die ausgewählte Bewegung des Siebschalengriffs, insbesondere durch die Bewegung des Siebschalengriffs von der Trageposition aus in die Fixierposition, wird also der Fixierabschnitt und/oder der Andrückabschnitt oder das Element, der/das zu dem Greifabschnitt beabstandet und folglich unterschiedlich zu diesem ist, in Richtung außerhalb der Sterilisiersiebschale bewegt und/oder deformiert.

Der Abstand des Greifabschnitts zu dem Fixierabschnitt und/oder Andrückabschnitt oder dem Element ist deswegen vorgesehen, da so eine Beeinträchtigung eines manuellen Handhabens vermieden wird. Durch den Abstand wird dem jeweiligen Abschnitt eine eigeständige Funktion zugeordnet. Dem Greifabschnitt wird die Funktion des händischen Ergreifens und dem Fixierabschnitt und/oder Andrückabschnitt oder dem Element wird die hierzu unterschiedliche Funktion eines Fixierens respektive Andrückens zugewiesen.

Dadurch also, dass der Siebschalengriff den Greifabschnitt zum manuellen Ergreifen und Handhaben aufweist, der über ein winklig zu dem Greifabschnitt ausgerichteten Verbindungssteg mit dem Lagerabschnitt verbunden und an der Siebschalenseitenwand gelagert, insbesondere angelenkt, ist, in Verbindung mit dem wenigstens einen Fixierabschnitt und/oder Andrückabschnitt oder Element, wird eine solche Konfiguration bereitgestellt, mittels derer sich die Sterilisiersiebschale durch Bewegung und/oder Deformation an eine externe, also außerhalb der Sterilisiersiebschale anordbare, (Gegen-) Kontaktfläche, Kontaktpunkt und/oder Kontaktsystem anlegen, andrücken, verbinden und/oder einen Abstand hierzu verringern kann.

Insbesondere weist die Sterilisiersiebschale in der Trageposition / Tragestellung des Siebschalengriffs eine geringere Breite und/oder Tiefe zweier abgewandter Abschnitte als Außenmaß als in der Fixierposition / Fixierstellung des Siebschalengriffs auf. Zwei definierte, einander abgewandte Kontaktpunkte bzw. (Kontakt-)Flächen der Sterilisiersiebschale können also bedingt durch die ausgewählte Bewegung und damit implizit durch die Position / Stellung des Siebschalengriffs in ihrer Distanz zueinander geändert werden. Bei vergrößerter Distanz können diese Kontaktpunkte bzw. Kontaktflächen, wenn die Sterilisiersiebschale etwa in einen Behälter oder in eine Containerwanne eingesetzt ist, an einer Innenwand anliegen (zumindest aber den umfänglichen Spalt verringern) und die Sterilisiersiebschale fixieren und sichern. Zumindest ein bewegbarer und/oder defomierbarer Kontaktpunkt bzw. eine Kontaktfläche bildet dabei den Fixierabschnitt und/oder Andrückabschnitt oder das Element. Wenn beispielsweise zwei oder mehr Kontaktpunkte bzw. Kontaktflächen durch die ausgewählte Bewegung und damit positionsabhängig des Siebschalengriffs in Richtung außerhalb der Sterilisiersiebschale bewegbar und/oder deformierbar sind, dann können diese auch jeweils den Fixierabschnitt und/oder Andrückabschnitt oder das Element bilden.

Insbesondere wird eine solche Sterilisiersiebschale zur Verfügung gestellt, die einen mit dem Siebschalengriff wirkverbundenen bzw. gekoppelten und bewegbaren, insbesondere schwenkbaren, Fixierabschnitt und/oder Andrückabschnitt oder Element aufweist, der/das insbesondere dafür angepasst ist mit einer Fläche, einem Gegen-Andrückabschnitt oder -element oder einer planen Ebene in Kontakt zu kommen. Vorzugsweie kann in der Trageposition / Tragestellung der Fixierabschnitt und/oder Andrückabschnitt oder Element in dem Siebschaleninnenvolumen, also innerhalb der Sterilisiersiebschale, angeordnet sein und gegenüber einer Außenseite der Siebschalenwandung nicht hervorzustehen, und durch die ausgewählte Bewegung in Richtung außerhalb der Sterilisiersiebschale bewegt und/oder deformiert werden, um insbesondere in der Fixierposition / Fixierstellung gegenüber der Außenseite der entsprechenden Siebschalenseitenwand oder zumindest eines Abschnitts der Außenseite nach außen vorzustehen. Auch kann der Fixierabschnitt und/oder Andrückabschnitt oder das Element bereits gegenüber der Außenseite hervorstehen und durch die ausgewählte Bewegung noch weiter in Richtung außerhalb der Sterilisiersiebschale bewegt und/oder deformiert werden. So kann eine, insbesondere größte, Abmessung der Sterilisiersiebschale in eine Richtung verändert, vorliegend erhöht, werden. Hierbei wird also vorzugsweise eine, insbesondere größte, Abmessung bzw. ein Abstand zwischen zwei einander abgewandten Außenflächen der Sterilisiersiebschale bzw. Teilabschnitte hiervon vergrößert, so dass ein (zweiseitiges) Verpressen (actio und reactio) in insbesondere einer Containerwanne bewirkt wird.

Mit noch anderen Worten ist weist die Sterilisiersiebschale wenigstens einen von dem Greifabschnitt beabstandeten Fixier- und/oder Andrückabschnitt oder Element auf, der/das an der wenigstens einen Siebschalenseitenwand gelagert ist und durch eine ausgewählte Bewegung des Siebschalengriffs von der Siebschalenseitenwand in Richtung außerhalb der Sterilisiersiebschale bewegbar und/oder deformierbar ist, um sich gegen eine Containerwanne anzulegen.

Durch diese Konfiguration der Sterilisiersiebschale kann einem Nutzer ein einfacher und effektiver Transport- und Kratzschutz bereitgestellt werden.

Die Richtung außerhalb der Sterilisiersiebschale ist dabei eine (jedwede mögliche) Richtung, welche von einem Siebschaleninnenvolumen weg zur äußeren Umgebung der Sterilisiersiebschale zeigt bzw. gerichtet ist. Mit anderen Worten zeigt die Richtung außerhalb der Sterilisiersiebschale von der Sterilisiersiebschale selbst weg. Die Richtung außerhalb der Sterilisiersiebschale weist insbesondere einen Richtungsanteil in die Breite und/oder Tiefe auf, um durch die Bewegung eine Änderung eines Anlagepunktes des Fixierabschnitts und/oder Andrückabschnitts oder Elements in der horizontalen Ebene zu bewirken. Insbesondere kann die Richtung außerhalb der Sterilisiersiebschale die Breiten- und/oder Tiefenrichtung sein.

Die ausgewählte Bewegung ist dabei eine spezielle Bewegung des Siebschalengriffs. Diese ausgewählte Bewegung kann beispielsweise eine Schwenkbewegung, eine Drehbewegung, eine translatorische Bewegung als auch eine Kombination aus diesen Bewegungen sein. Wichtig hierbei ist, dass der Fixierabschnitt und/oder Andrückabschnitt oder das Element aufgrund dieser ausgewählten Bewegung durch entsprechende Wirkverbindung kausal in Richtung außerhalb der Sterilisiersiebschale bewegbar und/oder deformierbar ist.

Der Begriff Trageposition / Tragestellung beschreibt hierbei eine (erste) Position / Stellung des Siebschalengriffs, in welcher ein manuelles Ergreifen und Tragen der Sterilisiersiebschale 1 für einen Nutzer gut möglich ist. Insbesondere erstreckt sich der Siebschalengriff gegenüber der Sterilisiersiebschale vertikal nach oben, so dass der Siebschalengriff von einem Nutzer einfach erfasst, vorzugsweise von oben umgriffen, und hängend gehalten werden kann.

Der Begriff Fixierposition / Fixierstellung / Spreizstellung beschreibt hingegen eine gegenüber der ersten Position unterschiedliche, zweite Position / Stellung des Siebschalengriffs, in welcher der Fixier- und/oder Andrückabschnitt oder das Element weiter in Richtung außerhalb der Siebschale bewegt und/oder deformiert wurde, sich insbesondere eine Abmessung zwischen zwei definierten Kontaktpunkten bzw. Kontaktflächen in einer Richtung gegenüber der Trageposition vergrößert hat und die Sterilisiersiebschale, insbesondere wenn diese in einer Containerwanne eines Containersystems eingesetzt ist, beispielsweise gegen ein Verrutschen, fixiert. Vorzugsweise ist die Fixierposition eine solche Position des bewegbaren Siebschalengriffs, in welcher sich der Siebschalengriff im Wesentlichen horizontal und weiter vorzugsweise nach innerhalb der Sterilisiersiebschale erstreckt.

Die Bezeichnungen Breite, Tiefe und Höhe werden nachstehend als Richtungsangaben bzw. Achsen eines kartesischen Koordinatensystems verwendet, wobei Breite und Tiefe, wie üblich, für eine Grundfläche, etwa den Siebschalenboden, verwendet wird. Die Begriffe "Oben" und "Unten" werden in Richtung der Höhe, die Begriffe "Links" und "Rechts" in Richtung der Breite und die Begriffe "Vorne" und "Hinten" in Richtung der Tiefe verwendet.

Weiter ist in der wenigstens einen Siebschalen(seiten)wand im Bereich des zumindest einen Siebschalengriffs eine Aussparung, insbesondere eine Öffnung, ausgebildet, durch welche der Fixier- und/oder Andrückabschnitt oder das Element durch die ausgewählte Bewegung des Siebschalengriffs in Richtung außerhalb der Sterilisiersiebschale bewegbar und/oder deformierbar ist und insbesondere in der Fixierposition gegenüber der Außenseite nach außen vorstehen kann. Durch die Aussparung wird eine Art geometrischer Kanal / Verbindung zwischen der Außenseite und dem Siebschaleninnenvolumen geschaffen, durch welche sich der Fixier- und/oder Andrückabschnitt oder das Element (bewegungs- und positionsabhängig des Siebschalengriffs) insbesondere hindurchbewegen kann. Vorzugsweise ist die Aussparung in Form einer rechteckigen Öffnung mit umfänglicher Umrandung (der Siebschalenwand) ausgebildet, welche sich insbesondere am oberen Randabschnitt der Siebschalenwand befindet. Weiter vorzugsweise weist die Sterilisiersiebschale an zwei sich gegenüberliegenden Siebschalenwänden jeweils eine Aussparung für jeweils einen Fixier- und/oder Andrückabschnitt oder Element auf. Insbesondere weist die wenigstens eine Siebschalenwand eine Vielzahl an Aussparungen für eine Vielzahl an Fixier- und/oder Andrückabschnitten oder Elementen auf.

Vorteilhafte Ausführungsformen sind in den Unteransprüchen beansprucht und werden insbesondere nachfolgend erläutert.

Gemäß einer bevorzugten Ausführungsform kann der Siebschalengriff nach Art eines zweiseitigen Hebels um ein Drehgelenk mit einer Drehachse schwenkbar angelenkt sein und der Siebschalengriff den zu der Drehachse beabstandeten Fixier- und/oder Andrückabschnitt oder das Element und den, insbesondere stabförmigen, zu der Drehachse beabstandeten Greifabschnitt zum manuellen Ergreifen des Siebschalengriffs aufweisen, wobei der Fixier- und/oder Andrückabschnitt oder das Element auf der einen Seite der Drehachse (erste Seite des Hebels) und der Greifabschnitt auf der anderen Seite der Drehachse (zweite Seite des Hebels) angeordnet ist, so dass eine Schwenkbewegung des Greifabschnitts eine gleichsinnige Schwenkbewegung des Fixier- und/oder Andrückabschnitts oder Elements um die Drehachse bewirkt. Mit anderen Worten ist der Siebschalengriff um eine Drehachse drehbar / schwenkbar an der Siebschalenwand angelenkt, wobei an/auf einer ersten Seite des zweiseitigen Hebels der Greifabschnitt angeordnet ist und auf einer zweiten Seite (der ersten Seite in Bezug auf die Drehachse gegenüberliegenden Seite) des zweiseitigen Hebels der Fixier- und/oder Andrückabschnitt oder das Element angeordnet ist. Sowohl der Greifabschnitt als auch der Fixier- und/oder Andrückabschnitt oder das Element sind zu der Drehachse beabstandet angeordnet. Der Greifabschnitt ist dabei dafür vorgesehen und ausgebildet, von einer Hand manuell ergriffen zu werden und die Sterilisiersiebschale tragen zu können. Wird nun der Siebschalengriff an seinem Greifabschnitt gehandhabt und geschwenkt, so bewirkt dies aufgrund der Geometrie des Siebschalengriffs als zweiseitiger Hebel automatisch eine Schwenkbewegung des Fixier- und/oder Andrückabschnitts oder Elements. Vorzugsweise ist der Greifabschnitt bügel- oder henkelförmig ausgebildet. Vorzugsweise liegt die Drehachse parallel zu (der Außenseite) der zugehörigen Siebschalenwand und/oder parallel zu einem Siebschalenboden. Alternativ kann auch ein Mechanismus, etwa eine Getriebeübersetzung zwischen dem Siebschalengriff und dem Fixier- und/oder Andrückabschnitt oder dem Element vorgesehen sein, etwa in Form eines Zahnstangengetriebes.

Gemäß einer weiteren bevorzugten Ausführungsform kann der Siebschalengriff in Form eines geraden Hebels ausgeführt sein, mit Anordnung von Griffabschnitt, Drehachse und Fixier- und/oder Andrückabschnitt oder Element in Richtung der Drehachse gesehen in gerader Verlängerung zueinander (ähnlich einer Wippe). Zusätzlich oder alternativ kann ein senkrechter Abstand zwischen dem Greifabschnitt (als Wirkungslinie der Kraft) und der Drehachse um ein Vielfaches, insbesondere ein 2-faches bis 6-faches, insbesondere 3-faches bis 4-faches größer als ein senkrechter Abstand zwischen dem Fixier- und/oder Andrückabschnitt oder Element und der Drehachse sein, um einen effektiven Hebelarm zu bilden. Alternativ kann der zweiseitige Hebel auch in Form eines geknickten Hebels, etwa L-förmig, ausgeführt sein, der den Fixier- und/oder Andrückabschnitt oder das Element ähnlich eines Exzenters verwendet.

Es kann zweckmäßig sein, wenn der Siebschalengriff eine Vielzahl von Fixier- und/oder Andrückabschnitten oder Elementen, vorzugsweise zwei oder drei, aufweist, welche insbesondere einen gleichen senkrechten Abstand zwischen dem Fixier- und/oder Andrückabschnitt oder Element einerseits und der Drehachse andererseits aufweisen und die vorzugsweise achsensymmetrisch, also zu einer Symmetrieebene senkrecht zu der Drehachse, angeordnet sind. Über beispielsweise eine Tiefe der Sterilisiersiebschale verteilt, können die Fixier- und/oder Andrückabschnitte oder Elemente dann eine Kraftübertragung aufteilen, dadurch lokale Spannungsspitzen vermeiden und die Kraft noch homogener über einen größeren Abschnitt aufgrund einer vergrößerten und verteilten Fläche übertragen und die Sterilisiersiebschale noch besser und sicherer fixieren.

Vorzugsweise kann der Fixier- und/oder Andrückabschnitt oder das Element einen Aufsatz mit Eigenelastizität aufweisen, der vorzugsweise Gummi, Silikon, insbesondere Schaumsilikon, und/oder Kunststoff als Material aufweist und insbesondere aus Gummi, Silikon, insbesondere Schaumsilikon, oder Kunststoff besteht, wobei der Aufsatz vorzugsweise lösbar/wechselbar mit dem Fixier- und/oder Andrückabschnitt oder Element koppelbar ist, insbesondere aufsteckbar und/oder aufclippbar ist. Damit kann der Fixier- und/oder Andrückabschnitt oder das Element im Bereich der äußeren Kontaktstelle ein anderes Material und Design als der restliche Siebschalengriff aufweisen, was u.a. einen Produktionsprozess verbessert. Der Aufsatz mit seiner Eigenelastizität ermöglicht einen Toleranzausgleich und eine Verspannung mit sicherer Positionierung der Sterilisierschalengriffe in insbesondere der Fixierposition. Wenn der Aufsatz lösbar ist, kann dieser als Verschleißprodukt schnell und einfach ausgetauscht werden. So kann der Aufsatz beispielsweise auf den Fixier- und/oder Andrückabschnitt oder das Element aufgesteckt und bei Bedarf, etwa nach einem vorgegebenen Zeitintervall oder einer Anzahl an Sterilisiervorgängen, wieder heruntergenommen werden. Vorzugsweise kann der Aufsatz gegenüber Stahl, insbesondere Edelstahl, einen Reibungskoeffizienten von größer 0,2, bevorzugt von über 0,5 besonders bevorzugt von über 0,7 aufweisen. Vorzugsweise kann der Aufsatz auf den Fixier- und/oder Andrückabschnitt oder das Element aufgespritzt sein.

Vorzugsweise weist der der Fixier- und/oder Andrückabschnitt oder das Element einen stabfömigen Aufsteckabschnitt mit einer D-förmigen Kontur bzw. Querschnitt (kreisförmigen Querschnitt mit abgeschnittener Seite) und der Aufsatz eine hierzu komplementäre D-förmige Durchgangsöffnung auf. So wird, wenn aufgesteckt, der Aufsatz geometriebedingt rotationsfest auf dem Aufsteckabschnitt gehalten.

Vorzugsweise kann der Aufsatz hülsenförmig gestaltet sein mit jedoch einer abgeschnittenen oder angefasten Außenseite, so dass abschnittsweise an der radialen Außenseite des Aufsatzes eine plane Anschlagsfläche ausgebildet ist (D-förmige Außenkontur, insbesondere auch D-förmige Innenkontur). Diese plane Anschlagsfläche der abgeschnittenen Außenseite steht dabei vorzugsweise in der Fixierposition, insbesondere als bereichsweise äußerste Fläche der Sterilisiersiebschale, in die Breiten- und/oder Tiefenrichtung hervor, wobei diese Anschlagsfläche vorzugsweise senkrecht zum Siebschalenboden steht (vertikal). Insbesondere ist die Anschlagsfläche in der Fixierposition parallel zu der Außenseite der Siebschalenwand (in diesem Bereich).

Gemäß einem Aspekt der Erfindung kann die Sterilisiersiebschale zumindest ein Siebschalengriff-Anbaumodul/Siebschalengriff-Beschlag aufweisen, welches das Lager, insbesondere das Gelenk, den in dem Lager bzw. Gelenk gelagerten bzw. angelenkten Siebschalengriff und eine Anbaustruktur aufweist. Das Siebschalengriff-Anbaumodul ist dafür vorgesehen und angepasst, als separates und fertig montiertes Modul mittels der Anbaustruktur formschlüssig und/oder stoffschlüssig an die wenigstens eine Siebschalenwand, insbesondere mittels einer Schraubenverbindung, einer Nietverbindung und/oder einer Schweißverbindung, angebaut/montiert/befestigt zu sein. So kann das Siebschalengriff-Anbaumodul unabhängig von der restlichen Sterilisiersiebschale separat gefertigt und ausgetauscht werden. Auch kann ein von dem Siebschalenboden und/oder Siebschalenwand unterschiedliches Material verwendet werden. Bevorzugt wird der Siebschalenboden und die Siebschalenwand einteilig hergestellt, etwa tiefgezogen, und es werden lediglich an einer oder zwei gegenüberliegenden Siebschalenseitenwänden die Siebschalengriff-Anbaumodule angebaut. Alternativ oder zusätzlich kann das Siebschalengriff-Anbaumodul auch mittels Kraftschluss, etwa wenn die Anbaustruktur als Klemmteil ausgebildet ist, kraftschlüssig montierbar sein. Insbesondere kann die Sterilisiersiebschale auf zwei sich gegenüberliegenden Seiten bzw. zwei einander gegenüberliegenden Siebschalenwänden das Siebschalengriff-Anbaumodul, insbesondere das gleiche Siebschalengriffanbaumodul, aufweisen.

Gemäß einem weiteren Aspekt der Erfindung kann die Sterilisiersiebschale an zwei sich gegenüberliegenden Rand-/Kantenabschnitten jeweils einen innenliegenden Siebschalengriff und einen zugehörigen Fixier- und/oder Andrückabschnitt oder Element aufweisen, um durch die ausgewählte Bewegung jeweils in abgewandte Richtungen außerhalb der Sterilisiersiebschale bewegbar und/oder deformierbar zu sein, insbesondere um in der Fixierposition gegenüber der Außenseite der Siebschalenwand in zwei abgewandte Richtungen nach außen vorzustehen. Dies hat den Vorteil, dass die Sterilisiersiebschale nur an definierten Stellen, nämlich den Fixier- und/oder Andrückabschnitten oder Elementen, vorzugsweise vorstehende, exponierte Kontaktpunkte bzw. Kontaktflächen hat und etwa mit einer Containerwanne in Kontakt kommt. So wird ein Verschleiß der restlichen Sterilisiersiebschale minimiert und eine definierte Krafteinleitung an zwei sich gegenüberliegenden Kontaktpunkten geschaffen, um die Sterilisiersiebschale, insbesondere in der Containerwanne, zu verspannen. Vorzugsweise liegen die beiden Drehachsen der Siebschalengriffe parallel zueinander. Vorzugsweise sind die beiden Siebschalengriffe Gleichbauteile, um eine Produktion zu vereinfachen.

Insbesondere ist der Siebschalenboden rechteckförmig, insbesondere mit abgerundeten Ecken, ausgestaltet und die umlaufenden Siebschalenseitenwände erstrecken sich entlang der Kanten des Siebschalenbodens und senkrecht zu diesem.

Insbesondere ist ein Gelenk, an dem der Siebschalengriff an der Siebschalenwand angelenkt ist, in Form eines Scharniers ausgestaltet. Vorzugsweise dient ein (um die Drehachse) gebogenes Blech als seitenwandfestes, erstes Teil des Scharniers und der stabförmige anscharnierte Lagerabschnitt des Siebschalengriffs mit zwei, sich beidseitig anschließenden abgebogenen Abschnitten, zwischen denen das gebogene Blech eingefasst ist, als zweites Teil des Scharniers (nach Art einer Stufe).

Vorzugsweise weist der Siebschalengriff eine im Wesentlichen trapezförmige Kontur auf, wobei ein gebogener Stab mit insbesondere kreisrundem Querschnitt diese Kontur bildet. Eine kurze Grundseite bildet dabei den Greifabschnitt, die lange Grundseite als Lagerabschnitt ist über den winklig zu dem Greifabschnitt ausgerichteten Verbindungssteg an der Siebschalenwand angelenkt und weist einen Versatz im Bereich des Gelenks und/oder im Bereich des Fixier- und/oder Andrückabschnitts oder des Elements auf, um den Fixier- und/oder Andrückabschnitt oder das Element senkrecht zu der Drehachse zu beabstanden.

Vorzugsweise weist der Siebschalengriff zwei separate Fixier- und/oder Andrückabschnitte oder Elemente auf, insbesondere mit zwei Aufsätzen, die in Tiefenrichtung zwischen Gelenken angeordnet sind und in Tiefenrichtung zueinander einen gleichen Abstand wie ein Abstand zu dem benachbarten Gelenk aufweisen. Insbesondere ist ein Abstand zwischen Drehgelenk-Fixierabschnitt-Fixierabschnitt-Drehgelenk (in dieser Reihenfolge) in der Breiten- und/oder Tiefenrichtung jeweils gleich.

Vorzugsweise weist die Sterilisiersiebschale an zwei gegenüberliegenden Siebschalenwänden jeweils einen in Tiefenrichtung gesehen mittig angeordneten Fixier- und/oder Andrückabschnitt oder Element auf. Alternativ kann die Sterilisiersiebschale auch diagonal sich gegenüberliegend angeordnete Fixier- und/oder Andrückabschnitte oder Elemente mit jeweils in Tiefenrichtung gesehen versetzt angeordneten Fixier- und/oder Andrückabschnitten oder Elementen aufweisen. Insbesondere kann die Sterilisiersiebschale genau zwei sich in Draufsicht gesehen diagonal an den Randabschnitten gegenüberliegende Fixier- und/oder Andrückabschnitte oder Elemente aufweisen. Vorzugsweise ist der Siebschalengriff ein Gleichbauteil.

Vorzugsweise kann der Fixierabschnitt und/oder Andrückabschnitt ein stabförmiger Abschnitt sein, der sich über mindestens 30%, bevorzugt über 50%, der Tiefe der Sterilisiersiebschale und in Richtung der Tiefe erstreckt.

Vorzugsweise kann der Siebschalengriff einen Nockenwellen-Abschnitt /nockenwellenartigen Abschnitt aufweisen, an dem an einer seiner Längsachse die Fixier- und/oder Andrückabschnitte oder Elemente und an der anderen Längsachse die anscharnierten Abschnitte des Siebschalengriffs vorgesehen sind. So stehen insbesondere nur die Fixier- und/oder Andrückabschnitte oder Elemente hervor und Aussparungen in der Siebschalenwand können minimiert werden. Auch können Aufsätze nicht entlang einer Längsachse verschoben werden.

Vorzugsweise kann das Element ein deformierbares Element, insbesondere ein elastisches Kissen sein, das vorzugsweise Elastomer als Material aufweist oder sogar insgesamt aus Elastomer besteht. Weiter bevorzugt kann das Kissen ein Gummikissen oder ein Silikonkissen sein. Besonders bevorzugt kann das Silikonkissen im gesamten aus Silikon bestehen (Vollsilikon). Vorzugsweise kann bei einer ausgewählten Bewegung des Siebschalengriffs, insbesondere bei der Bewegung von der Trageposition in die Fixierposition, durch einen Mechanismus mittels zielgerichtetem Quetschen das deformierbare Element in Richtung nach außerhalb der Siebschale deformiert werden. Insbesondere kann der Siebschalengriff bei seiner ausgewählten Bewegung geometriebedingt an einer Seite oder Stelle des Kissens aufdrücken und so das Kissen in eine andere Lage zwängen, um das deformierbare Element gezielt in Richtung außerhalb der Sterilisiersiebschale zu deformieren.

Gemäß einer Ausführungsform kann der Siebschalengriff derart an der Siebschalenwand gelagert sein, dass er translatorisch in eine Richtung bzw. entlang einer Achse bewegbar ist. Vorzugsweise kann der Siebschalengriff derart an der Siebschalenwand gelagert sein, dass dieser eine translatorische Bewegung in die vertikale Richtung (Höhenrichtung; nach oben/unten) durchführen kann. Insbesondere kann der Siebschalengriff, vorzugsweise der Verbindungssteg, einen mit der zugehörigen Lagerung zusammenwirkenden Anschlag aufweisen, um die translatorische Bewegung in zumindest eine Richtung zu begrenzen. Vorzugsweise weist der Verbindungssteg zwei Anschläge auf, um die translatorische Bewegung des Siebschalengriffs zwischen einer ersten Position, insbesondere einer Trageposition, und einer zweiten Position, insbesondere einer Fixierposition, zu begrenzen. An der Siebschalenwand kann, vorzugsweise im Bereich einer Aussparung, ein verformbares, elastisches Kissen gelagert sein, das mit dem Siebschalengriff zusammenwirkt. Die Sterilisiersiebschale ist dabei vorzugsweise derart konfiguriert, dass bei einer translatorischen ausgewählten Bewegung des Siebschalengriffs, der Siebschalengriff auf eine vordefinierte Stelle des elastischen Kissens presst / drückt und dieses Kissen entgegen seiner elastischen Vorspannkraft in Richtung außerhalb deformiert. Insbesondere steht der Siebschalengriff in der Trageposition außer Kontakt und in der Fixierposition in direktem Kontakt mit dem elastischen Kissen. Vorzugsweise ist der Siebschalengriff U-Förmig gestaltet mit zwei zueinander parallel erstreckenden Abschnitten als die Verbindungsstege (lange Seiten des U) und einem zwischen diesen angeordneten Greifabschnitt (kurze Seite des U). Dieser U-förmige Siebschalengriff steht vorzugsweise mit seinen beiden parallelen Verbindungsstegen jeweils durch eine Führungsöffnung oder ein längliches Bohrloch in vorzugsweise der Höhenrichtung oder durch zwei zueinander beabstandete, aber koaxiale (Führungs-)Öffnungen oder Bohrlöcher, so dass eine translatorische Führung des Griffs (ähnlich eines Bolzens) ausgebildet ist. Das elastische Kissen ist dabei insbesondere jeweils in Tiefenrichtung gesehen auf gleicher Position wie der Verbindungssteg, jedoch in Breitenrichtung gesehen weiter nach außerhalb der Siebschale angeordnet, so dass bei einer Bewegung des Siebschalengriffs, insbesondere einer Abwärtsbewegung, der Verbindungssteg direkt auf das elastische Kissen drückt und dieses aufgrund der geometrischen Anordnung nach außerhalb presst. Vorzugsweise kann das elastische Kissen, insbesondere zusammen mit dem Lager, gegenüber dem Innenvolumen der Siebschale von einem Gehäuse zumindest teilweise umschlossen sein, um so den Mechanismus gegen störende Einflüsse, etwa verrutschte Instrumente, zu schützen.

Das elastische Kissen weist insbesondere im Längsschnitt gesehen ein Dreieck als Grundform, mit vorzugsweise abgerundeten Ecken, auf. Diese Dreiecksform bildet dabei die Grundfläche eines Blocks, welcher sich insbesondere in Tiefenrichtung der Siebschale erstreckt. Insbesondere ist das elastische Kissen derart an der Siebschalenwand befestigt, gehalten oder gelagert, dass in einem nicht deformierten Zustand eine (etwa lange) Seite bzw. Seitenfläche des dreieckigen Kissens in Richtung der Höhe und/oder parallel zum zugehörigen Siebschalenseitenwandabschnitt angeordnet ist. Auf diese Weise kann im nicht deformierten Zustand die Siebschale gut in eine Containerwanne eingesetzt werden. Wird der Griff mittels der ausgewählten Bewegung verschoben, so presst der Verbindungssteg in Folge auf die andere, schräge Seite des dreieckigen Kissens, welche als Art Rampe dient, und zwängt dadurch das Kissen, welches insbesondere siebschaleninnenwandseitig an seinem oberen Ende befestigt ist, nach außerhalb. Hierbei wird das Kissen um eine Achse in Tiefenrichtung elastisch verdreht und deformiert. Wird der Griff mit dem bzw. den Verbindungssteg(en) wieder nach oben bewegt, so entfällt der Anpressdruck wieder und das Kissen verformt sich in seinen ursprünglichen, nicht deformierten Zustand zurück. Alternativ kann das Kissen auch um eine Achse drehbar gelagert sein und sich über seine Schwerkraft wieder in seinen Ausgangszustand nach innerhalb bewegen.

Vorzugsweise ist das Lager ein umgeformtes Blechbauteil mit zumindest zwei parallelen Blechabschnitten, die sich in die Breiten- und Tiefenrichtung erstrecken und jeweils koaxiale und insbesondere kongruente Öffnungen oder Bohrlöcher, insbesondere mit einer Öffnungsachse in die Höhenrichtung, aufweisen, so dass ein Verbindungssteg des Griffs in diesen (Führungs-)Öffnungen translatorisch bewegbar ist. Vorzugsweise ist die Öffnung nicht rotationssymmetrisch ausgebildet, um ein Verdrehen und Verkanten zu verhindern und etwa einen Anschlag zu ermöglichen.

Die Aufgabe der Erfindung wird hinsichtlich eines gattungsgemäßen Sterilisiersiebschalensystems erfindungsgemäß dadurch gelöst, dass das Sterilisiersiebschalensystem einen Sterilisiersiebschalendeckel und eine erfindungsgemäße Sterilisiersiebschale aufweist. Insbesondere kann der Sterilisiersiebschalendeckel eine im Wesentlichen platten- oder gitterartige Grundform aufweisen, welche eine Oberseite definiert, und vorzugsweise derart an die Sterilisiersiebschale angepasst sein, um zusammen mit den Siebschalengriffen einen Verschlussmechanismus bereitzustellen.

Die Aufgabe der Erfindung wird hinsichtlich eines gattungsgemäßen Containersystem erfindungsgemäß dadurch gelöst, dass das Containersystem einen Container/Sterilcontainer und eine erfindungsgemäße Sterilisiersiebschale aufweist. Der Sterilcontainer, welcher auch ein beliebiger, herkömmlicher bzw. bekannter Container sein kann, weist dabei eine Containerwanne auf und kann ferner vorzugsweise einen zur Containerwanne passenden Containerdeckel aufweisen, um ein Innenvolumen des Containers gegenüber einer Umgebung abzugrenzen. Auch kann der Sterilcontainer auf Seiten der Containerwanne und/oder auf Seiten des Containerdeckels einen Filter aufweisen. Die Sterilisiersiebschale ist insbesondere dafür angepasst in die Containerwanne des Containers einsetzbar zu sein, wobei der Fixier- und/oder Andrückabschnitt oder das Element in der Fixierposition an der Innenwand der Containerwanne unmittelbar anliegt (mit dieser in Kontakt steht) und die Sterilisiersiebschale gegen die Containerwanne (in zumindest einer Richtung) verspannt und so die Sterilisiersiebschale in dem Container lagefixiert und sichert. Die Sterilisiersiebschale weist also kleinere Außenabmessungen als die Innenabmessung der Containerwanne auf, um in diese hineingesetzt zu werden. Dadurch bildet sich insbesondere umfänglich ein (kleiner) Spalt zwischen der Innenwand der Containerwanne und der Seitenwand der Sterilisiersiebschale aus, was eine Bewegung der Sterilisiersiebschale in der Containerwanne zulassen würde. Wird oder werden nun jedoch der/die Siebschalengriff(e) ausgewählt bewegt, so bewegen und/oder deformieren sich die Fixier- und/oder Andrückabschnitte oder Elemente in Richtung außerhalb der Siterilisiersiebschale und in Folge in Richtung zu der Containerwanne und kontaktiert/en aufgrund der Wirkverbindung mit dem/den Fixier- und/oder Andrückabschnitt(en) oder Element(en) unmittelbar die Innenwand des Sterilcontainers. Wenn etwa an zwei sich gegenüberliegenden Randabschnitten der Siebschalenwand ein Siebschalengriff vorgesehen ist, so (ver-)spreizen diese mit den Fixier- und/oder Andrückabschnitten oder Elementen gewissermaßen die Sterilisiersiebschale gegen die gegenüberliegenden Innenwände der Containerwanne und fixieren diese in dem Container. Die Containerwanne und die Sterilisiersiebschale sind geometrisch also so aufeinander abgestimmt, dass diese Spreiz-, Andrück-, bzw. Verspannfunktion verwirklicht wird.

### Kurzbeschreibung der Figuren

Die vorliegende Erfindung wird nachfolgend anhand von bevorzugten Ausführungsformen unter Bezugnahme der begleitenden Figuren näher erläutert. Es zeigen:
- Fig. 1: eine perspektivische Ansicht einer erfindungsgemäßen Sterilisiersiebschale gemäß einer bevorzugten Ausführungsform, bei welcher beide Siebschalengriffe sich in der Trageposition befinden,
- Fign. 2 und 3: eine perspektivische Ansicht der Sterilisiersiebschale aus Fig. 1, bei welcher einer der beiden Siebschalengriffe durch eine ausgewählte Bewegung in die Fixierposition geschwenkt wurde,
- Fig. 4: eine Frontansicht der Sterilisiersiebschale aus Fign. 1 bis 3,
- Fig. 5: eine Längsschnittansicht der Sterilisiersiebschale aus Fign. 1 bis 4, die in einem erfindungsgemäßen Sterilcontainersystem gemäß einer bevorzugten Ausführungsform eingesetzt ist, wobei sich beide Siebschalengriffe in der Trageposition befinden,
- Fig. 6: eine Längsschnittansicht der Sterilisiersiebschale und des Sterilcontainersystems aus Fig. 5, wobei sich beide Siebschalengriffe in der Fixierposition befinden,
- Fig. 7: eine Draufsicht auf die Sterilisiersiebschale und das Sterilcontainersystem aus Fig. 6,
- Fign. 8 und 9: eine perspektivische Teilansicht einer Sterilisiersiebschale gemäß einer weiteren bevorzugten Ausführungsform,
- Fig. 10: eine perspektivische Teilansicht der Sterilisiersiebschale aus Fign. 8 und 9, welche in Containerwanne eingesetzt ist und bei der sich ein Siebschalengriff in der Fixierposition befindet,
- Fig. 11: eine perspektivische Teilansicht einer erfindungsgemäßen Sterilisiersiebschale einer weiteren bevorzugten Ausführungsform bei der ein Fixierabschnitt pro Seite vorgesehen ist,
- Fig. 12: eine Draufsicht der Sterilisiersiebschale einer weiteren bevorzugten Ausführungsform, welche in einer Containerwanne eingesetzt ist und bei der die Siebschalengriffe in der Fixierposition sind, und welche einen Fixierabschnitt pro Seite aufweist, welche diagonal angeordnet sind,
- Fig. 13: eine perspektivische Ansicht einer erfindungsgemäßen Sterilisiersiebschale einer weiteren bevorzugten Ausführungsform, mit einem mittigen Fixierabschnitt pro Seite
- Fig. 14: eine perspektivische Ansicht einer erfindungsgemäßen Sterilisiersiebschale einer weiteren bevorzugten Ausführungsform, welche multiple, hier drei, Fixierabschnitte pro Seite aufweist,
- Fig. 15: eine perspektivische Ansicht einer erfindungsgemäßen Sterilisiersiebschale einer weiteren bevorzugten Ausführungsform mit einem stabförmigen Fixierabschnitt je Seite,
- Fig. 16: eine schematisch Längsschnittteilansicht einer erfindungsgemäßen Sterilisiersiebschale und eines Sterilcontainersystems einer weiteren bevorzugten Ausführungsform, mit einem elastischen Kissen, und
- Fig. 17: eine perspektivische Ansicht einer erfindungsgemäßen Sterilisiersiebschale einer weiteren bevorzugten Ausführungsform,
- Fig. 18: eine weitere perspektivische Ansicht der Sterilisiersiebschale aus Fig. 17;
- Fig. 19: eine Seitenansicht der Sterilisiersiebschale aus Fign.17 und 18, und
- Fig. 20: eine Längsschnittansicht der Sterilisiersiebschale aus Fign. 17 bis 19.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Beschreibung bevorzugter Ausführungsformen

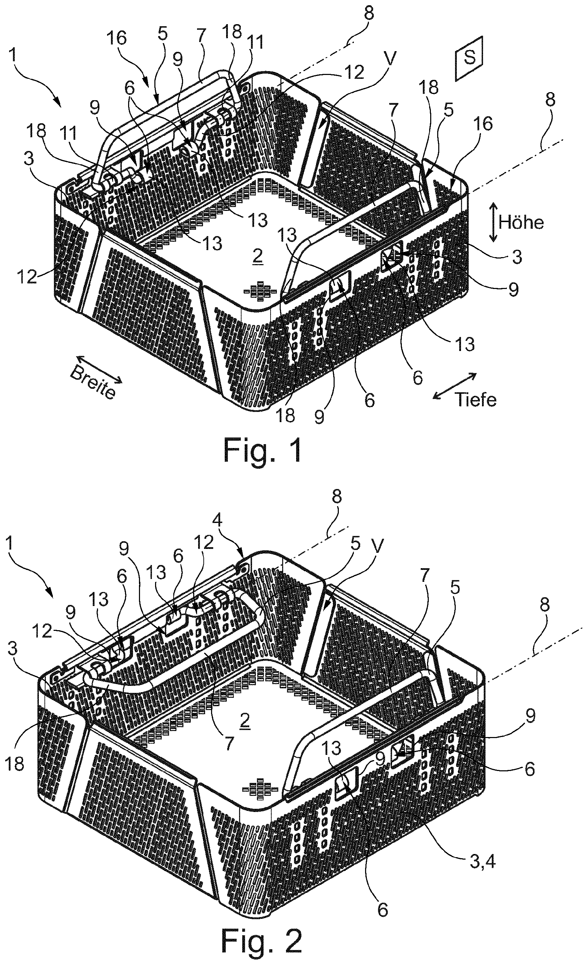

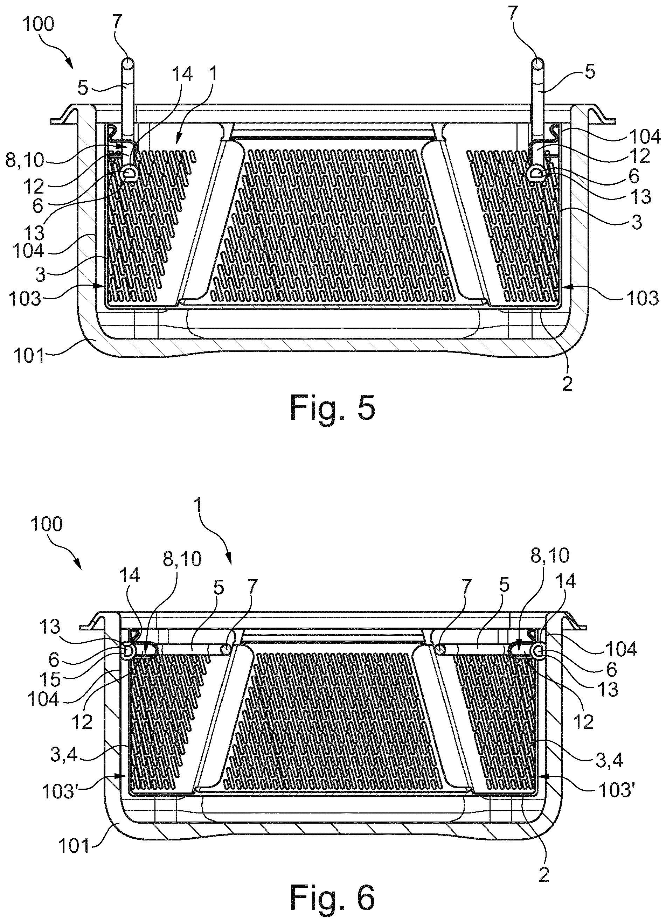

Figuren 1 bis 7 zeigen eine erfindungsgemäße Sterilisiersiebschale 1 (nachfolgend nur Siebschale genannt) gemäß einer ersten bevorzugten Ausführungsform, welche zum Einsetzen in und Entnehmen aus einem Containersystem vorgesehen und ausgebildet ist und vorliegend in dem erfindungsgemäßen (Steril-)Containersystem 100 einer bevorzugten Ausführungsform eingesetzt ist (siehe Fign. 5 bis 7) bzw. einsetzbar ist.

Die Siebschale 1 weist einen ebenen/planen, gitterförmigen, quadratischen Siebschalenboden 2 mit abgerundeten Ecken auf, welcher vollumfänglich von vier gitterförmigen Siebschalenwänden 3 mit umfänglich gleicher Höhe, die sich senkrecht zum Siebschalenboden 2 erstreckt, umschlossen ist. Der Siebschalenboden 2 und die Siebschalenwände 3 definieren dabei ein Siebschaleninnenvolumen V bzw. ein Siebschaleninneres, in welchem beispielsweise medizinische oder chirurgische Instrumente aufgenommen und für eine Sterilisation angeordnet werden können.

Die Sterilisiersiebschale 2 weist ferner zwei an sich gegenüberliegenden oberen Randabschnitten der Siebschalenwand 3 innenliegende, schwenkbare, henkelförmige Siebschalengriffe 5 auf, die jeweils unabhängig voneinander in zumindest eine Trageposition / Tragestellung und in eine Fixierposition / Fixierstellung schwenkbar sind. Jeder der beiden Siebschalengriffe 5 hat einen Greifabschnitt 7, der über einen winklig zu dem Greifabschnitt 7 ausgerichteten Verbindungssteg 18 mit einem Lagerabschnitt / einem anscharniertern Bereich 11 des Siebschalengriffs 7 verbunden ist.

Erfindungsgemäß hat die Siebschale 1 an beiden Siebschalengriffen 5 (nachfolgend als Griff bezeichnet) jeweils einen von dem Greifabschnitt 7 beabstandeten, wirkverbundenen / gekoppelten und bewegbaren, insbesondere schwenkbaren, Fixierabschnitt 6, der durch die ausgewählte Bewegung des Griffs 5 von der Siebschalenseitenwand 3 in Richtung außerhalb der Siebschale 1 bewegbar und/oder deformierbar ist.

In dieser Ausführungsform ist der Fixierabschnitt 6 in der Trageposition in dem Siebschaleninnenvolumen V angeordnet und steht gegenüber einer Außenseite 4 der Siebschalenwand 3 nicht hervor, wobei bei der ausgewählten Bewegung des Griffs 5 von der Trageposition in die Fixierposition der Fixierabschnitt 6 in Richtung außerhalb der Sterilisiersiebschale bewegt wird und in der Fixierposition des Griffs 5 gegenüber der Außenseite 4 der Siebschalenwand 3 nach außen hervorsteht, um sich anzulegen. Konkret sind in dieser Ausführungsform die beiden Griffe 5 Gleichbauteile und jeweils als zweiseitiger Hebel ausgeführt, der neben dem (schwenkbaren) Greifabschnitt 7 zum manuellen Ergreifen des Griffs 5 auch den Fixierabschnitt 6 als Strukturabschnitt aufweist und um eine Drehachse 8 schwenkbar ist.

Hierdurch ist also ein Strukturabschnitt des einteilig ausgeführten Griffes 5, nämlich der Fixierabschnitt 6, derart konfiguriert, dass in der Trageposition, in welcher die henkelförmigen Griffe 5 im Wesentlichen senkrecht in die Höhe stehen und von einem Nutzer für ein Herausholen und Hineinstellen der Siebschale 1 gut manuell von oben ergriffen werden können, der Fixierabschnitt 6 in dem Siebschaleninnenvolumen liegt und gegenüber der zugehörigen Siebschalenwand 3 bzw. der Außenfläche 4 nicht hervorsteht. In einem eingesetzten Zustand, in dem die Siebschale 1 in einer Containerwanne 101 eingesetzt ist, bildet sich ein umfänglicher Spalt 103 zwischen der Innenwänden 104 der Containerwanne 101 und den Seitenwänden 3 bzw. den durch diese definierten Außenflächen 4 aus (siehe Fign. 5 bis 7). Dieser umfängliche Spalt 103 ermöglicht eine gute Handhabung der Siebschale 1, insbesondere ein leichtes Herausnehmen und ein Verschieben, aber auch eine Verschiebung der Siebschale 1 in Breiten- und Tiefenrichtung.

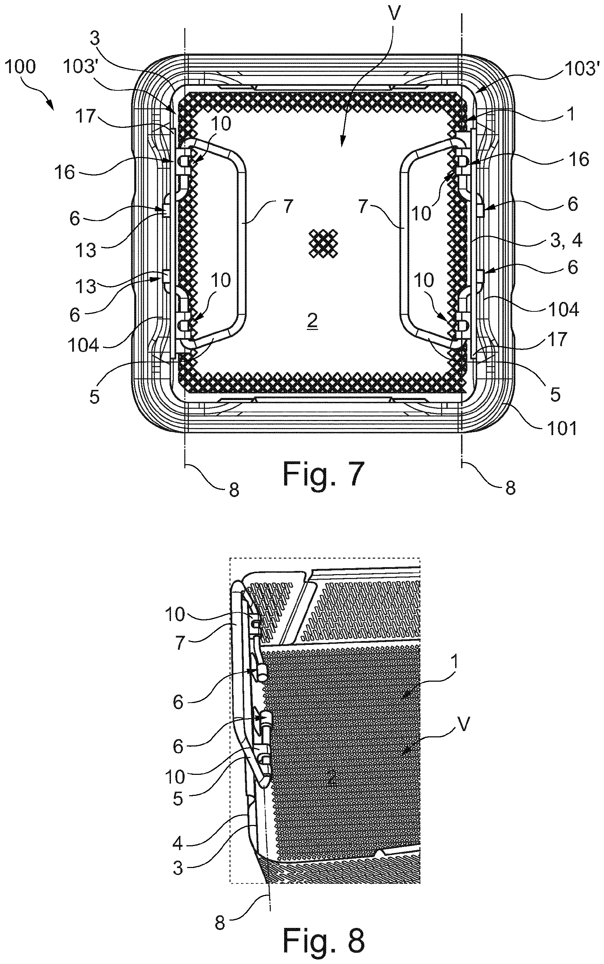

Wird nun im eingesetzten Zustand ein Griff 5 (siehe Fig. 2 linke Seite) bzw. beide Griffe 5 (siehe Fign. 6, 7) durch die ausgewählte Bewegung von der Trageposition in die Fixierposition um die Drehachse 8 geschwenkt, so bewirkt die zweiseitige Hebelkonfiguration des Griffs 5, dass der Greifabschnitt 7 nach innen in das Siebschalenvolumen V hineingeschwenkt wird, während gleichzeitig sowie gleichsinnig der dem Greifabschnitt 7 gegenüberliegende Fixierabschnitt 6 um die Drehachse 8 aus einer Aussparung 9 in der zugehörigen Siebschalenwand 3 von der Siebschalenwand 3 in Richtung außerhalb der Siebschale 1 herausgeschwenkt wird und gegenüber der Außenseite 4 (in die Breitenrichtung) hervorsteht. Hierdurch wird gewissermaßen eine Abmessung bzw. Distanz zwischen den zwei einander abgewandten Außenseiten 4 bzw. zwei gegenüberliegenden Siebschalenwänden 3 vergrößert. Dies bewirkt, dass der zuvor vollumfängliche Spalt 103 nicht mehr vollumfänglich, sondern gewissermaßen als unterbrochener Spalt 103' vorliegt. Die Fixierabschnitte 6 unterbrechen in der Fixierposition den Spalt 103' und liegen an einer Innenwand 104 der Containerwanne 101 unmittelbar an bzw. kontaktieren diese.

In der Fixierposition wird also mittels der (veränderbaren) vergrößerten Breite durch die sich gegenüberliegenden Fixierabschnitte 6, wie nachstehend auch noch detailliert erläutert, die Siebschale 1 in der Containerwanne 101 in zumindest einer Richtung, hier die Breitenrichtung (parallel zum Siebschalenboden 3) fixiert und gesichert. Durch diese erfindungsgemäße Konfiguration der Siebschale 1 wird ein Verrutschen im Container 101, 102 verhindert und ein einfacher und effizierter Transport- sowie Kratzschutz bereitgestellt. Ferner wird eine gute Handhabung der Siebschale 1 gewährleistet und die Siebschale 1 lässt sich in der Trageposition leicht in die Containerwanne 101 einführen und auch wieder herausholen. Ein Umschalten zwischen einer Handhabungsstellung und einer Fixierungsstellung erfolgt in besonders einfacher Weise.

Der Fixierabschnitt 6 ist in Tiefenrichtung gesehen zwischen den zwei anscharniertern Bereichen 11 angeordnet. Alternativ kann der Fixierabschnitt natürlich auch gegenüber den anscharnierten Bereichen 11 in Tiefenrichtung gesehen außerhalb dieser bzw. nach außen angeordnet sein.

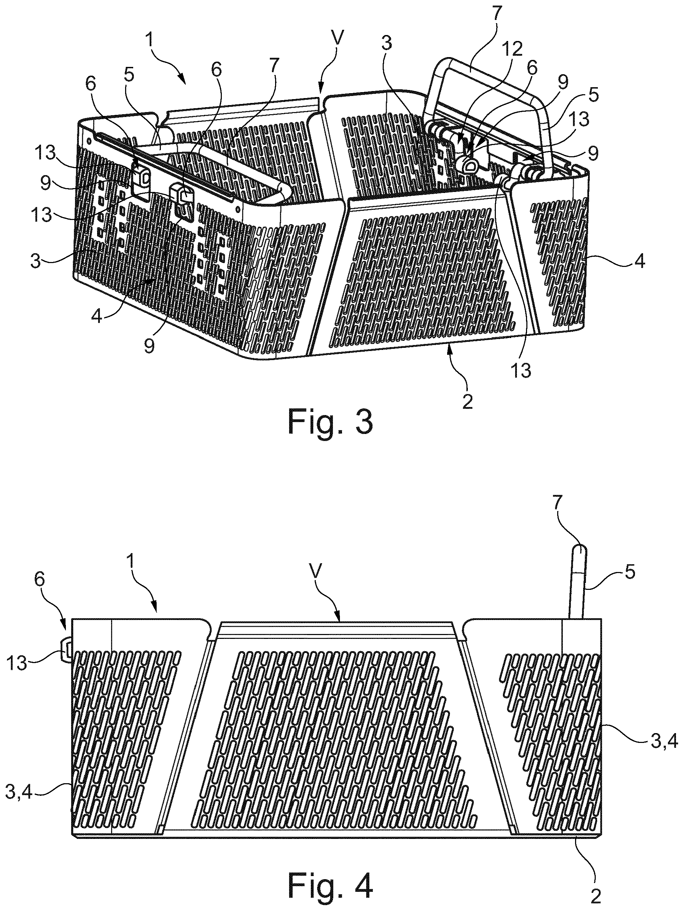

Die an den vier Seiten/Kanten des Siebschalenbodens 2 jeweils ebene, vertikale und gitterförmige Siebschalenwand 3 mit jeweils bogenförmigem Übergang (abgerundete Eckkante) an den Ecken definieren eine dem Siebschaleninnenvolumen V bzw. dem Siebschalenboden 2 abgewandte Außenseite / Außenfläche 4 bzw. in dieser Ausführungsform vier Außenflächen 4 mit zwei mal zwei einander abgewandten Außenflächen 4 (der zwei sich gegenüberliegenden Siebschalenwände 3), welche die Sterilisiersiebschale 1 gegenüber der Umgebung zu den Seiten hin abgrenzt. Wie insbesondere in Fig. 4 gezeigt, bilden die einander abgewandten Außenflächen 4 jeweils eine Ebene parallel zueinander, die sich beide senkrecht zum Siebschalenboden 2 in die Höhe und entlang des Siebschalenbodens 2 in die Tiefe erstreckt.

In der ersten Ausführungsform der Fign. 1 bis 7 ist die Siebschale 1 symmetrisch zu einer vertikalen (ersten) Symmetrieebene S gestaltet, wobei auf jeder Seite ein Siebschalengriff 5 vorgesehen ist. Im Folgenden ist es daher ausreichend, wenn nur eine Seite der Sterilisiersiebschale 1 detailliert beschrieben wird. Ferner ist in der ersten Ausführungsform die Siebschale 1 sogar auch zu einer weiteren vertikalen Symmetrieebene, welche senkrecht zu der ersten Symmetrieebene S steht, symmetrisch. Die Siebschale 1 weist also vier (spiegel-)symmetrische Viertel auf.

Die henkelförmigen, schaleninnenseitigen Griffe 5 an zwei sich gegenüberliegenden Seitenflächen der Seitenwand 3 sind mittels eines Drehgelenks 10 nach Art eines Scharniers um die Drehachse 8 drehbar bzw. schwenkbar anscharniert. Die Drehachse 8 liegt parallel zu der Außenfläche 4 der Siebschalenwand 3 und zu dem Siebschalenboden 2. Die Griffe 5 können so von einer im Wesentlichen vertikal nach oben zeigenden Trageposition / Ergreif-Stellung / Ausklapp-Stellung nach innen in das Siebschaleninnenvolumen V oder umgekehrt geschwenkt werden. Die henkelförmigen Griffe 5 sind an einem der Siebschalenboden 2 abgewandten und in den Fign. 1 bis 7 gesehen oberen Abschnitt der zugehörigen Siebschalenwand 3 in dem Drehgelenk 10 angelenkt.

Der Griff 5 ist ein umgeformter zylinderförmiger Metallstab mit kreisrundem Querschnitt. Durch die Umformung bildet der Metallstab nun eine im Wesentlichen trapezförmige Kontur. Die kurze Grundseite des Trapezes formt den stabförmigen/zylinderförmigen Greifabschnitt 7 aus, der sich stets in Richtung der Tiefe der Siebschale 1 (also in allen Positionen / Stellungen stets parallel zu dem Siebschalenboden 2) erstreckt. Die lange Grundseite des Trapezes ist nahe den zwei kurzen Verbindungsstegen 18 als Schenkel anscharniert und mittig unterbrochen. Konkret ist der anscharnierter, stabförmiger Bereich / Lagerabschnitt 11 der langen Grundkante parallel zu dem Greifabschnitt 7 geformt und macht nach diesem Bereich jeweils einen Z-förmigen oder stufenförmigen Absatz / Abknickung / Versatz 12 von dem Greifabschnitt 7 und der Drehachse 8 weg. Dieser stufenförmige Absatz 12 weist also zwei parallele stabförmige Abschnitte (der anscharnierte Bereich 11 sowie der Fixierabschnitt 6) und einen zwischen diesen angeordneten schräg stehenden Abschnitt auf. Der endständige Abschnitt des Absatzes 12, der parallel zu dem Greifabschnitt 7, zu der Drehachse 8 und zu dem Siebschalenboden 2 ist, bildet dabei den Fixierabschnitt 6 des Griffs 5.

Durch den stufenförmigen Absatz 12 ist der Fixierabschnitt 6 senkrecht zu der Drehachse 8 beabstandet und bildet die zweite Seite des zweiseitigen Hebels aus. Der gesamte Griff 5 ist so geformt bzw. der Stab als Griff 5 so umgeformt, dass dieser in einer Ebene liegt. Wie auch in der Längsschnittansicht in Fig. 5 gezeigt, ist durch den Griff 5 ein gerader zweiseitiger Hebel (eine Gerade zwischen dem Fixierabschnitt 6, der Drehachse 8 und dem Greifabschnitt 7, in dieser Reihenfolge, in einem Längsschnitt in Breiten-/Höhenrichtung gesehen) mit lediglich in der Tiefe versetzter Anordnung (unterschiedliche Tiefenpositionen) gebildet. Anders ausgedrückt, ist in Verlängerung der Verbindung des Greifabschnitts 7 und der Drehachse 8, der Fixierabschnitt 6 auf der anderen Seite bezüglich der Drehachse 8 ausgebildet. In dieser Ausführungsform ist ein senkrechter (kürzester) Abstand zwischen dem Greifabschnitt 7 und der Drehachse 8 für eine Hebelwirkung etwa ein vierfaches größer ein senkrechter Abstand zwischen der Drehachse 8 und dem Fixierabschnitt 6.

An dem Fixierabschnitt 6 ist ein Aufsatz 13 aus Silikon, insbesondere Schaumsilikon, mit einer Eigenelastizität aufgesteckt. Durch den Aufsatz 13 werden Toleranzen zwischen der Innenwand 104 der Containerwanne 101 und den Fixierabschnitten 6 ausgeglichen. Der Aufsatz 13 ist hülsenförmig mit jedoch einer abgeflachten planen (radialen) Innen- und Außenseite gestaltet (D-förmig). Ebenso ist der stabförmige Griff 5 beim Fixierabschnitt 6 an einer dem Greifabschnitt 7 abgewandten Seite plan abgeschliffen, so dass auch dieser eine D-förmige Außen-Kontur 14 aufweist. Der D-förmige, hülsenförmige Aufsatz 13 ist auf den passenden komplementären D-förmigen Aufsteckabschnitt aufgesteckt, wodurch der Rotationsfreiheitsgrad des Aufsatzes 13 gesperrt ist. Zudem bildet der Aufsatz 13 in der Fixierposition jeweils eine senkrechte plane Anschlagsfläche / Fixierfläche 15 parallel zu der Außenseite 4 der zugehörigen Siebschalenwänden 3, um flächig an der Innenwand 104 anzuliegen. Der Reibungskoeffizient zu der Innenwand 104 ist durch den Aufsatz 13 aus Silikon erhöht. Auch kann der Aufsatz 13 als Verschleißteil einfach getauscht werden.

Jeder der beiden Griffe 5 weist in dieser Ausführungsform zwei Fixierabschnitte 6 mit gleichem senkrechtem Abstand zu der Drehachse 8 auf, welche in Tiefenrichtung gesehen zwischen den Drehgelenken 10 angeordnet sind. Jeder der insgesamt vier Fixierabschnitte 6 hat in der Siebschalenwand 3 jeweils eine zugehörige Aussparung 9 in Form der quadratischen Öffnung. Wird der Griff 5 durch die ausgewählte Bewegung in die Fixierposition geschwenkt, so stehen die Fixierabschnitte 6 mit den Aufsätzen 13 jeweils durch die Aussparungen 9 in Breitenrichtung gegenüber der Außenseite 4 in zwei abgewandte Richtungen hervor und bilden die größte Abmessung der Siebschale 1 (siehe Fig. 7). Die Fixierabschnitte 6 verspannen so die Sterilisiersiebschale 1 in der Containerwanne 101 in Breitenrichtung. Durch die Aufsätze 13 aus Silikon mit einhergehendem hohen Reibungskoeffizienten und Anschlagsfläche 15 wird zudem eine Fixierung in Tiefenrichtung als auch in Höhenrichtung erreicht.

Sowohl in dem Siebschalenboden 2 als auch in der Siebschalenwand 3 sind eine Vielzahl an quadratischen Öffnungen und Längsschlitze ausgestanzt, um eine Fluidverbindung zwischen der Innenseite und der Außenseite 4 herzustellen und so die Außenseite 4 insbesondere für Wasserdampf durchdringbar zu gestalten. Als Material weisen der Siebschalenboden 2 und die Siebschalenwand 3 Aluminium und/oder Edelstahl auf.

In dieser Ausführungsform weist die Siebschale 1 ein Siebschalengriff-Anbaumodul 16 mit einer Anbaustruktur 17 auf. Das Siebschalengriff-Anbaumodul 16 mit dem Drehgelenk 10, dem Griff 5 und der Anbaustruktur ist dabei als separates Modul hergestellt und anschließend mit der restlichen Siebschale 1, also dem Siebschalenboden 2 und der Siebschalenwand 3, vernietet. Das Drehgelenk 10 ist jeweils als ein um die Drehachse 8 umgebogenes Blech ausgeführt, durch welches der anscharnierte Bereich 11 des Griffs 5 hindurchsteht. Das Siebschalengriff-Anbaumodul 16 kann mit etwa unterschiedlichen Materialen zur restlichen Siebschale 1 ausgestaltet und separat vorab montiert werden, was einen Produktionsprozess verbessert.

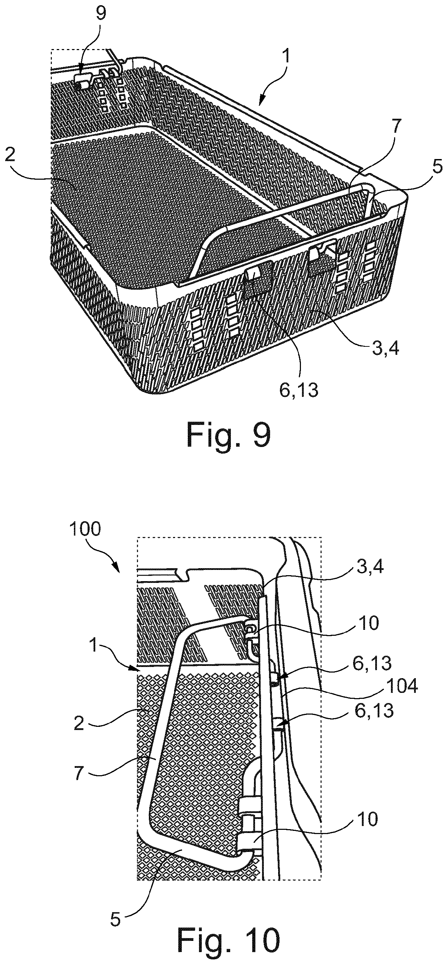

Fig. 8 bis 10 zeigen eine weitere, zweite bevorzugte Ausführungsform des Siebkorbs 1 mit einem rechteckigen Siebschalenboden 2.

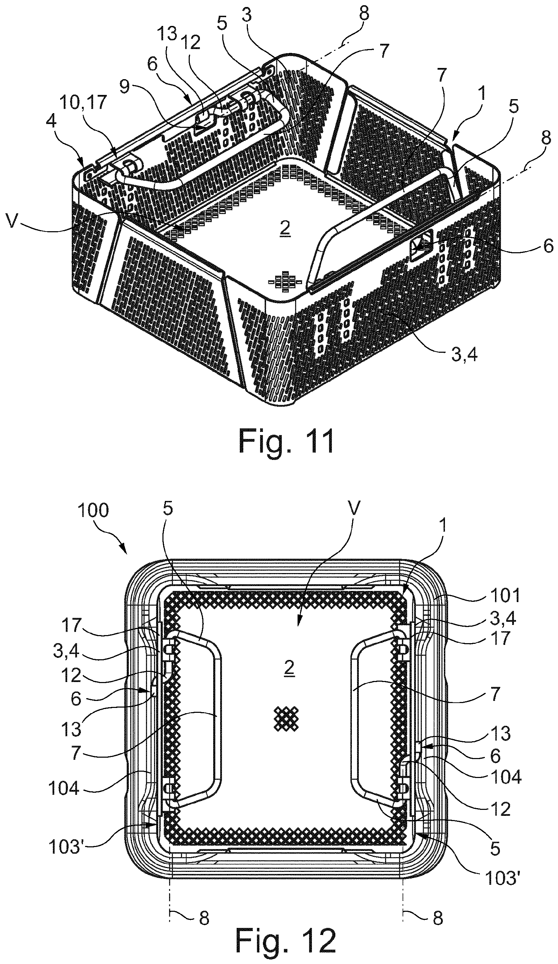

Fig. 11 zeigt eine weitere, dritte bevorzugte Ausführungsform der Siebschale 1. Die zwei an gegenüberliegenden Siebschalenwänden 3 vorgesehen Griffe 5 sind Gleichbauteile. Im Unterschied zur ersten Ausführungsform ist jedoch an jedem Griff 5 nur ein Fixierabschnitt 6 ausgebildet. Der zweite stufenförmige Absatz mit dem zugehörigen Fixierabschnitt ist entfallen. Der eine Fixierabschnitt 6 ist in Tiefenrichtung gesehen nicht mittig des Griffs 5, sondern versetzt zur Mitte angeordnet ist. In der Siebschalenwand 3 ist jeweils nur eine zugehörige quadratische Öffnung als Aussparung 9 vorgesehen. Beide Fixierabschnitte 6 liegen auf gleicher Position in Tiefenrichtung.

Fig. 12 zeigt eine weitere, vierte bevorzugte Ausführungsform der Siebschale 1 und eines Containersystems 100 mit zwei sich diagonal gegenüberliegenden Fixierabschnitten 6. Beide Griffe 5 sind wieder gleich geformt und an gegenüberliegenden Siebschalenwänden 3 vorgesehen. Der Griff 5 ist der gleiche wie aus der Ausführungsform aus Fig. 11 mit dem Unterschied, dass der eine Griff 5 um eine vertikale Achse um 180° gedreht und dann an die Siebschalenwand 3 angelenkt wurde, so dass sich die Fixierabschnitte 6 und die Aussparungen 9 in einer Draufsicht wie in Fig. 12 diagonal gegenüberliegen.

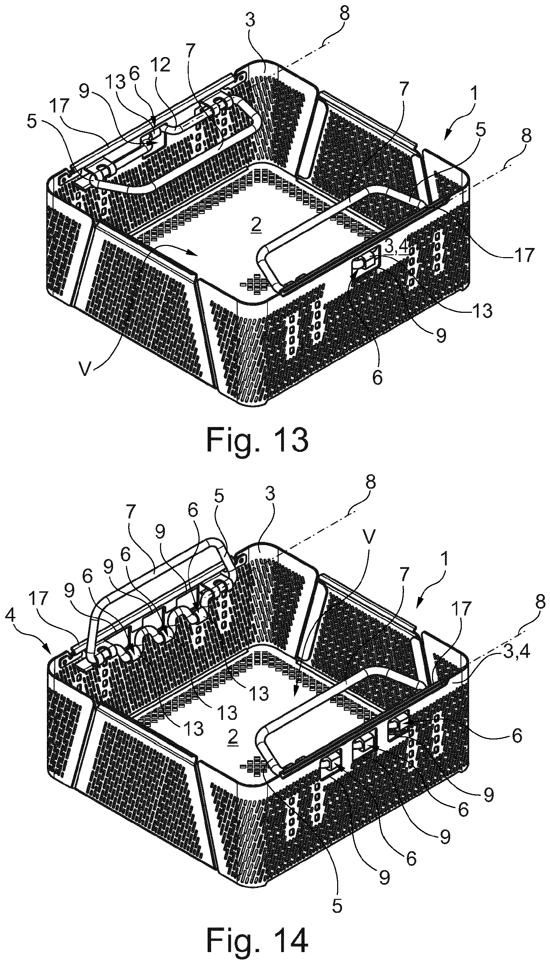

Fig. 13 zeigt eine weitere, fünfte bevorzugte Ausführungsform der Siebschale 1, in welcher der henkelförmige, trapezförmige Griff 5 an seiner langen Grundseite nun durchgängig stabförmig gestaltet ist und lediglich ein mittiger stabförmiger Abschnitt als stufenförmiger Absatz 12 hiervon zu der Drehachse 8 gegenüber dem Greifabschnitt 7 zurückversetzt ist, ähnlich einer Ausbuchtung. Der mittige Abschnitt als Fixierabschnitt 6 weist wieder den Aufsatz 13 auf, der aufclippbar oder aufgespritzt ist.

Fig. 14 zeigt eine weitere, sechste bevorzugte Ausführungsform der Siebschale 1, in welcher jeder Griff 5 drei in Tiefenrichtung gesehen gleichverteilte Fixierabschnitte 6 mit jeweils Aufsätzen 13 und gleichem senkrechtem Abstand zur Drehachse 8 aufweist. Die Siebschale 1 weist an den zwei sich gegenüberliegenden Siebschalenwänden 3 entsprechend drei komplementäre quadratische Öffnungen als Aussparungen 9 auf. Die lange Grundseite des trapezförmigen, henkelförmigen Griffs 5 ist dabei nockenwellenartig mit drei stufenförmigen Absätzen 12 gestaltet.

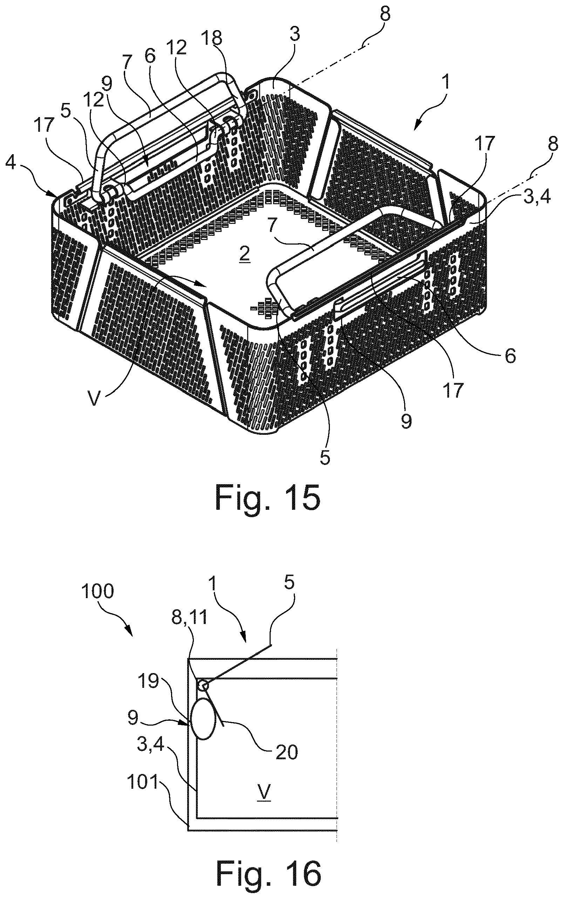

Fig. 15 zeigt eine weitere, siebte bevorzugte Ausführungsform der Siebschale 1, welche zwei gegenüberliegende Griffe 5 ohne Aufsatz aufweist. Die lange Grundseite der trapez- und henkelförmigen Griffen 5 ist durchgängig ausgeführt und durch zwei stufenförmige Absätze 12 jeweils direkt neben den zwei Drehgelenken 10 beginnend gegenüber der Drehachse 8 zurückversetzt. Alternativ kann man auch sagen, dass der anscharnierte Bereich 11 des Griffs gegenüber der langen Grundseite des Trapezes zur kurzen Grundseite hin versetzt ist. Der lange stabförmige Abschnitt zwischen den anscharnierten Bereichen 11 bildet dabei den Fixierabschnitt 6. Die zwei gegenüberliegenden Aussparungen 9 in den Siebschalenwänden 3 sind in dieser Ausführungsform als in Tiefenrichtung langgezogene rechteckige Öffnung ausgeführt, welche sich etwa in 50% der Tiefe der Siebschale 1 erstreckt. An dem in der Fixierposition vorstehenden, langen, stabförmigen Fixierabschnitt 6 wurde eine in Breitenrichtung äußere vertikale plane Anschlagsfläche 15 vorgesehen, so dass der Fixierabschnitt 6 eine D-förmige Kontur aufweist. Die plane Anschlagsfläche 15 ist in der Fixierposition parallel zu der jeweiligen Außenseite 4 und bildet die in Breitenrichtung gesehen äußerste, in Tiefenrichtung langgezogene, (Kontakt-)Fläche am oberen Randabschnitt der Siebschale 1.

Fig. 16 zeigt in einer schematischen Längsschnitt-Teilansicht eine weitere, siebte bevorzugte Ausführungsform einer erfindungsgemäßen Siebschale 1, welche als Fixier- und/oder Andrückabschnitt oder Element ein elastisches Kissen, hier ein Gummikissen 19, aufweist, welches sich bei einer ausgewählten Bewegung des Siebschalengriffs 5 von der Trageposition in die Fixierposition durch einen Mechanismus mittels zielgerichtetem Quetschen nach außerhalb deformiert. Das Gummikissen 19 ist dabei an der Siebschalenseitenwand 3 fixiert und steht in der Fixierposition durch die Aussparung 9 gegenüber der Außenseite 4 abschnittsweise zu der Containerwanne 101 hervor. Als Mechanismus ist vorliegend vorgesehen, dass der Griff 5 als zweiseitiger, L-förmiger Hebel einen um 90° angewinkelten Quetscharm 20 aufweist, der in der Fixierposition nach innen bzw. unten in die Siebschale 1 zeigt. Bei der ausgewählten Bewegung des Griffs 5 in die Fixierposition drückt der Quetscharm 20 geometriebedingt an einer Seite/Stelle des Gummikissens 19 auf, um bei fortlaufender ausgewählter Bewegung des Griffs 5 Druck aufzubauen und so das Gummikissen 19 gezielt in Richtung außerhalb zu Containerwanne 101 hin zu deformieren, um dort anzuliegen. Insbesondere ist die Siebschale 1 dafür ausgebildet, dass der Griff 5 in der Fixierposition fixierbar ist. Durch die Deformation in Richtung außerhalb wird die Siebschale 1 in der Containerwanne 101 in Breitenrichtung verspannt.

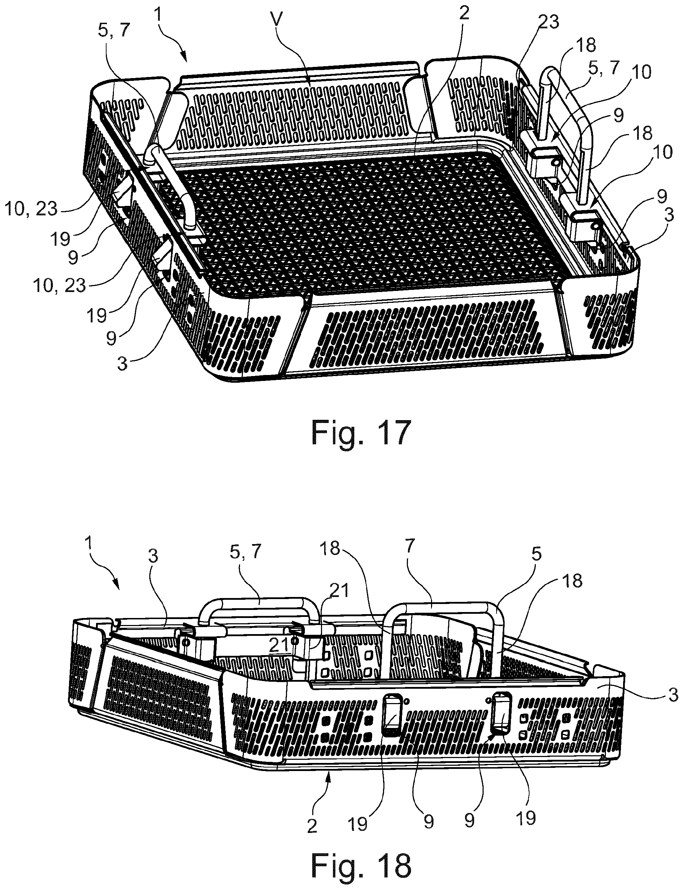

Die Fign. 17 bis 20 zeigen eine weitere, achte bevorzugte Ausführungsform der Siebschale 1, in welcher als fixierende Elemente elastische Vollsilikon Kissen 19 vorgesehen sind. Der (umgedreht) U-förmige Griff 5 ist siebschaleninnenwandseitig an zwei Lagern 10 translatorisch gelagert und kann nach oben in die Trageposition und nach unten in die Fixierposition geschoben werden. Das Lager 10 ist dabei in Form eines umgeformten Blechbauteils mit zwei parallel zueinander angeordneten Blechabschnitten 22 ausgebildet, in welchen koaxiale und kongruente Führungsöffnungen 23 ausgebildet sind, deren gemeinsame Achse sich in Richtung der Höhe erstreckt. Jede Führungsöffnung 23 weist als Kontur eine Kreisform mit abgeschnittener Seite auf bzw. eine Öffnungskontur mit gerader Grundseite und einem anschließenden teilkreisförmigen Bogen. In je (zwei) Führungsöffnungen 23 steht jeweils ein Verbindungssteg 18 des Griffs 5 hinein. Der U-förmige Griff 5 in Form eines gebogenen, zylinderförmigen Stabs, weist dabei einen kreisförmigen Querschnitt auf, wobei an sogenannten Laufabschnitten der Verbindungsstege 18 (Abschnitte, die in dem Lager 10 geführt werden und mit diesen interagieren) entlang der Längsachse eine zu der Führungsöffnung 23 komplementäre Fase bzw. gerade, plane Abfräsung eingebracht ist. Durch diese Konfiguration ergeben sich oberhalb und unterhalb der Fase jeweils ein Anschlag 24 (kreisrunder Querschnitt steht gegenüber dem angefasten Laufabschnitt über) so dass die Schiebebewegung des Griffs zwischen der Trageposition und der Fixierposition begrenzt ist. Man kann auch sagen, dass der Griff 5 über die Verbindungsstege 18 und die Führungsöffnungen nach Art verschieblich gelagerter Bolzen eine Linearbewegung ausführen kann

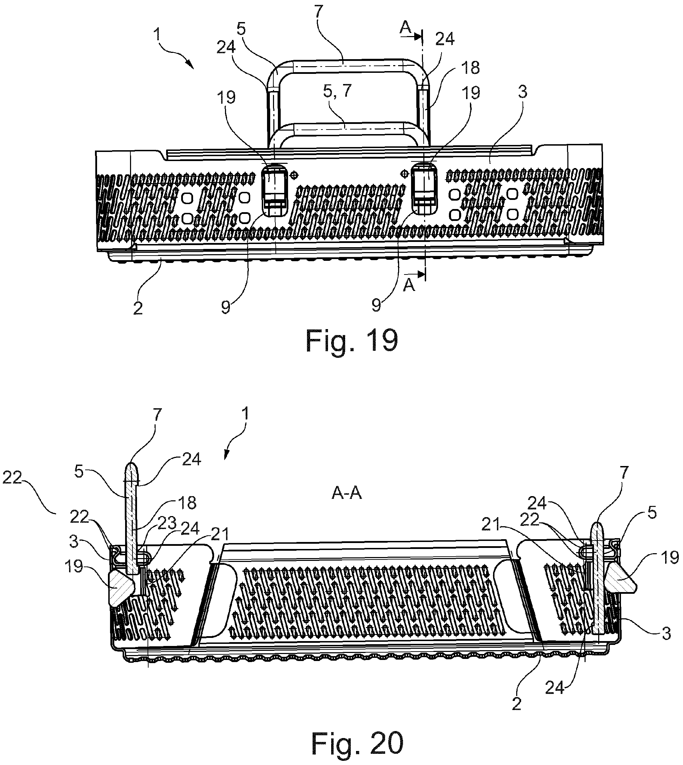

Ferner sind siebschaleninnenwandseitig jeweils in gleicher Tiefe wie der zugehörige Verbindungssteg 18, jedoch in Breitenrichtung gesehen weiter nach außerhalb der Siebschale 1, die Kissen 19 angeordnet. Die Kissen 19 sind an einem siebschaleninnenwandseitig vorgesehenen Gehäuse 21 an ihren jeweils oberen Abschnitten mit diesem befestigt. Das Gehäuse 21 umschließt den Mechanismus und grenzt ihn gegenüber dem Innenvolumen V ab. Entscheidend ist, dass die Kissen 19 dabei an ihrem oberen Ende fest mit dem Gehäuse 21 und damit mit der Siebschalenseitenwand 3 verbunden (sprich lokal fixiert) sind. Die Kissen 19 weisen in einer Vorderansicht bzw. Längsschnittansicht, wie insbesondere in Fig. 20 ersichtlich, eine dreieckige Grundform auf und erstrecken sich blockförmig in die Tiefenrichtung. In einem nicht deformierten Zustand (Griff 5 ist in die Trageposition nach oben hochgezogen; s. rechte Seite in Fign. 17, 18) steht bereits eine der drei Seitenflächen des jeweiligen Kissens 19 durch die Aussparung 9 hindurch und damit leicht gegenüber der Siebschalenwand 3 hervor und erstreckt sich parallel zu der Siebschalenwand 3. In einer alternativen nicht gezeigten Ausführungsform kann diese Seitenfläche auch bündig mit der Siebschalenseitenwand 3 oder innerhalb der Siebschale 1 angeordnet sein und nicht hervorstehen.

Wird nun der Griff 5 von der Trageposition (siehe rechte Seite Fig. 17, 18) in die Fixierposition (siehe linke Seite Fig. 17, 18) geschoben, so stößt bei dieser ausgewählten Bewegung jeweils ein unteres freies Ende des Verbindungsstegs 18 jeweils auf eine (andere) schräge Seite des zugehörigen dreiecksförmigen (keilförmigen) Kissens 19. Diese schräge Seite dient als Art Rampe und verdrängt in Folge der Bewegung das elastische Kissen 19 entgegen seiner elastischen Rückstellkraft und seinem Eigengewicht nach außerhalb durch die Aussparung 9. Die Kissen 19 werden sozusagen wie eine starre Schaukel mit elastischem Drehgelenk nach außen geschwenkt bzw. deformiert. Auf diese Weise können mittels der ausgewählten Bewegung des Griffs 5 durch die Aussparung 9 die Kissen 19 in Richtung außerhalb der Siebschale 1 deformiert werden. Sie stehen gegenüber der Siebschalenseitenwand 3 abschnittsweise (keilförmig) vor. Die Kissen 19 liegen dann an der Innenwand der Containerwanne an und fixieren die Siebschale 1. Wie etwa aus Fig. 20 ersichtlich, können sich die Kissen 19 in der Fixierposition auch nicht in die Breitenrichtung zurück ins Innere der Siebschale 1 bewegen. Der Verbindungssteg 18, der sich nur in Richtung der Höhe bewegen lässt und rückseitig an den Kissen 19 anliegt, beschränkt geometrisch den Freiheitsgrad in Breitenrichtung.

Wird der Griff 5 nun aus der Fixierposition entgegen seinem Eigengewicht und der Reibungskraft der anliegenden Kissen 19 wieder in die Trageposition angehoben, wird in Folge der Anpressdruck des Griffs 5 auf die Kissen 19 aufgehoben und die Kissen 19 bewegen sich in ihren nicht deformierten Ausgangszustand zurück. Die Siebschale 1 ist dann nicht mehr in der Containerwanne fixiert und lässt sich aus dieser entnehmen.

### Bezugszeichenliste

- 1: Sterilisiersiebschale
- 2: Siebschalenboden
- 3: Siebschalen(seiten)wand
- 4: Außenseite
- 5: Siebschalengriff
- 6: Fixierabschnitt
- 7: Greifabschnitt
- 8: Drehachse
- 9: Aussparung
- 10: Lager / Drehgelenk
- 11: Lagerabschnitt / anscharnierter Bereich des Siebschalengriffs
- 12: Stufenförmiger Absatz
- 13: Aufsatz
- 14: D-förmige Kontur
- 15: Anschlagsfläche
- 16: Siebschalengriff-Anbaumodul
- 17: Anbaustruktur
- 18: Verbindungssteg
- 19: Silikonkissen / Gummikissen
- 20: Quetscharm
- 21: Gehäuse
- 22: Blechabschnitt
- 23: Führungsöffnung
- 24: Anschlag
- 100: Sterilcontainersystem
- 101: Containerwanne
- 102: Containerdeckel
- 103: Spalt
- 103': unterbrochener Spalt
- 104: Innenwand
- V: Siebschaleninnenvolumen
- S: Symmetrieebene

## Patentansprüche

1. Sterilisiersiebschale (1), die zum Einsetzen in und Entnehmen aus einer Containerwanne (101) vorgesehen und ausgebildet ist, wofür an wenigstens einer Siebschalenseitenwand (3) wenigstens ein, vorzugsweise bügelförmiger oder henkelförmiger, Siebschalengriff (5) bewegbar, insbesondere schwenkbar, gelagert ist, der einen Greifabschnitt (7) hat, der über einen winklig zu dem Greifabschnitt (7) ausgerichteten Verbindungssteg (18) mit einem Lagerabschnitt (11) verbunden ist, an welchem der Siebschalengriff (5) an einem siebschalenwandseitigen Lager (10) gehalten ist und in zumindest eine Trageposition und in eine Fixierposition bewegbar, insbesondere schwenkbar ist, und die Steriliersiebschale (1) wenigstens einen von dem Greifabschnitt (7) beabstandeten Fixier- und/oder Andrückabschnitt (6) oder Element aufweist, der/das an der wenigstens einen Siebschalenseitenwand (3) gelagert ist und durch eine ausgewählte Bewegung des Siebschalengriffs (5) von der Siebschalenseitenwand (3) in Richtung außerhalb der Sterilisiersiebschale (1) bewegbar und/oder deformierbar ist, um sich gegen eine Containerwanne (101) anzulegen, darurch gekennzeichnet, dass in der wenigstens einen Siebschalenseitenwand (3) im Bereich des zumindest einen Siebschalengriffs (5) eine Aussparung (9) ausgebildet ist, durch welche der Fixier- und/oder Andrückabschnitt (6) oder das Element durch die ausgewählte Bewegung des Siebschalengriffs (5) in Richtung außerhalb der Sterilisiersiebschale (1) bewegbar und/oder deformierbar ist und insbesondere in der Fixierposition gegenüber der Siebschalenseitenwand (3) nach außen vorsteht.

2. Sterilisiersiebschale (1) nach Anspruch 1, **dadurch gekennzeichnet, dass** der Siebschalengriff (5) nach Art eines zweiseitigen Hebels ausgeführt und um ein Drehgelenk (10) mit einer Drehachse (8) schwenkbar angelenkt ist und der Siebschalengriff (5) den zu der Drehachse (8) beabstandeten Fixier- und/oder Andrückabschnitt (6) oder das Element und den, insbesondere stabförmigen, zu der Drehachse beabstandeten Greifabschnitt (7) zum manuellen Ergreifen des Siebschalengriffs (5) aufweist, wobei der Fixier- und/oder Andrückabschnitt (6) oder das Element auf der einen Seite der Drehachse (8) und der Greifabschnitt auf der anderen Seite der Drehachse (8) angeordnet ist, so dass eine Schwenkbewegung der Greifabschnitts (7) eine gleichsinnige Schwenkbewegung des Fixier- und/oder Andrückabschnitts (6) oder des Elements um die Drehachse bewirkt.

3. Sterilisiersiebschale (1) nach Anspruch 2, **dadurch gekennzeichnet, dass** der Siebschalengriff (5) in Form eines geraden Hebels ausgeführt ist und/oder ein senkrechter Abstand zwischen dem Greifabschnitt (7) und der Drehachse (8) um ein Vielfaches, insbesondere ein 2-faches bis 6-faches, insbesondere 3-faches bis 4-faches größer als ein senkrechter Abstand zwischen dem Fixier- und/oder Andrückabschnitt (6) oder dem Element und der Drehachse (8) ist, um einen effektiven Hebelarm zu bilden.

4. Sterilisiersiebschale (1) nach einem der vorangegangenen Ansprüche,
**dadurch gekennzeichnet, dass** der Siebschalengriff (5) eine Vielzahl von Fixier- und/oder Andrückabschnitte (6) oder Elemente, vorzugsweise zwei oder drei, aufweist, welche einen gleichen senkrechten Abstand zwischen dem Fixier- und/oder Andrückabschnitt (6) oder dem Element einerseits und der Drehachse (8) andererseits aufweisen und die vorzugsweise achsensymmetrisch entlang der Drehachse (8) angeordnet sind.

5. Sterilisiersiebschale (1) nach einem der vorangegangenen Ansprüche,
**dadurch gekennzeichnet, dass** der Fixier- und/oder Andrückabschnitt (6) oder das Element einen Aufsatz mit Eigenelastizität aufweist, der vorzugsweise Gummi, Silikon und/oder Kunststoff als Material aufweist und insbesondere aus Gummi, Silikon oder Kunststoff besteht, und der Aufsatz vorzugsweise lösbar an den Fixierabschnitt (6) ankoppelbar, insbesondere auf den Fixierabschnitt (6) aufsteckbar und/oder aufclippbar ist.

6. Sterilisiersiebschale (1) nach einem der vorangegangenen Ansprüche,
**dadurch gekennzeichnet, dass** die Sterilisiersiebschale (1) zumindest ein Siebschalengriff-Anbaumodul (16) aufweist, welches das Lager (10), den in dem Lager (10) gelagerten Siebschalengriff (5) und eine Anbaustruktur (17) aufweist, das als separates Modul mittels der Anbaustruktur (17) formschlüssig und/oder stoffschlüssig an der Siebschalenwand (3), insbesondere mittels einer Schraubenverbindung, einer Nietverbindung und/oder einer Schweißverbindung, befestigt ist.

7. Sterilisiersiebschale (1) nach Anspruch 6, **dadurch gekennzeichnet, dass** die Sterilisiersiebschale (1) an zwei sich gegenüberliegenden Rand-/Kantenabschnitten jeweils einen innenliegenden Siebschalengriff (5) und einen zugehörigen Fixier- und/oder Andrückabschnitt (6) oder Element aufweisen, um bei der ausgewählten Bewegung jeweils in abgewandte Richtungen außerhalb der Sterilisiersiebschale bewegbar und/oder deformierbar zu sein, insbesondere um in der Fixierposition gegenüber der Außenseite (4) der Siebschalenwand (3) in zwei abgewandte Richtungen nach außen vorzustehen.

8. Sterilisiersiebschalensystem mit einem Sterilisiersiebschalendeckel und einer Sterilisiersiebschale (1) nach einem der Ansprüche 1 bis 7.

9. Containersystem (100) mit einem Container (101, 102) und mit einer Sterilisiersiebschale (1) nach einem der Ansprüche 1 bis 7, wobei die Sterilisiersiebschale (1) in eine Containerwanne (101) des Containers einsetzbar ist und derart angepasst ist, dass in der Fixierposition der Fixier- und/oder Andrückabschnitt (6) oder das Element an der Innenwand (104) der Containerwanne (101) anliegt und die Sterilisiersiebschale (1) gegen die Containerwanne (101) verspannt und so die Sterilisiersiebschale (1) in dem Container (101, 102) lagefixiert und sichert.

## Claims

1. A sterilization sieve basket (1), which is provided and configured for insertion into and removal from a container tray (101), for which purpose at least one, preferably bowshaped or arched, sieve basket handle (5) is movably, in particular pivotably, mounted on at least one sieve basket side wall (3), which has a gripping portion (7) which is connected, via a connection web (18) oriented at an angle to the gripping portion (7), to a bearing portion (11) on which the sieve basket handle (5) is held on a bearing (10) on the side of the sieve basket wall (3) and is movable, in particular pivotable, into at least one carrying position and into a fixed position, and the sterilization sieve basket (1) has at least one fixing portion and/or press-on portion (6) or element spaced from the gripping portion (7) and supported on the at least one sieve basket side wall (3) and movable and/or deformable by selected movement of the sieve basket handle (5) away from the sieve basket side wall (3) in the direction toward the outside of the sterilization sieve basket (1) in order to abut against a container tray (101), **characterized in that** a recess (9) is formed in the at least one sieve basket side wall (3) in the area of the at least one sieve basket handle (5), through which the fixing portion and/or press-on portion (6) or the element can be moved and/or deformed by the selected movement of the sieve basket handle (5) in the direction outside of the sterilization sieve basket (1) and, in particular, projects outward in the fixed position relative to the sieve basket side wall (3).

2. The sterilization sieve basket (1) according to claim 1, **characterized in that** the sieve basket handle (5) is configured in the manner of a two-sided lever and is pivotably hinged about a pivot joint (10) with a rotational axis (8), and the sieve basket handle (5) comprises the fixing portion and/or press-on portion (6) or the element spaced from the rotational axis (8) and the, in particular rod-shaped, gripping portion (7) spaced from the rotational axis for manual gripping of the sieve basket handle (5), wherein the fixing portion and/or press-on portion (6) or element is arranged on one side of the rotational axis (8) and the gripping portion is arranged on the other side of the rotational axis (8), so that a pivoting movement of the gripping portion (7) causes a pivoting movement of the fixing portion and/or press-on portion (6) or element about the rotational axis in the same direction.

3. The sterilization sieve basket (1) according to claim 2, **characterized in that** the sieve basket handle (5) is configured in the form of a straight lever, and/or a perpendicular distance between the gripping portion (7) and the rotational axis (8) is greater by a multiple, in particular 2-fold to 6-fold, in particular 3-fold to 4-fold greater than a perpendicular distance between the fixing portion and/or press-on portion (6) or element and the rotational axis (8) in order to form an effective lever arm.

4. The sterilization sieve basket (1) according to one of the preceding claims,
**characterized in that** the sieve basket handle (5) comprises a plurality of fixing portions and/or press-on portions (6) or elements, preferably two or three, which have an equal perpendicular distance between the fixing portion and/or press-on portion (6) or element on the one hand and the rotational axis (8) on the other hand and which are preferably arranged axisymmetrically along the rotational axis (8).

5. The sterilization sieve basket (1) according to one of the preceding claims,
**characterized in that** the fixing portion and/or press-on portion (6) or the element comprises an attachment with inherent elasticity, which preferably comprises rubber, silicone and/or plastic as material and in particular consists of rubber, silicone or plastic, and the attachment is preferably detachably coupleable to the fixing portion (6), in particular is attachable and/or clippable onto the fixing portion (6).

6. The sterilization sieve basket (1) according to one of the preceding claims,
**characterized in that** the sterilization sieve basket (1) comprises at least one extension module of the sieve basket handle (16), which has the bearing (10), the sieve basket handle (5) mounted in the bearing (10) and an extension structure (17), and wherein the extension module is fastened to the sieve basket wall (3), in particular via a screw connection, a riveted connection and/or a welded connection, as a separate module by means of the extension structure (17) in a form-fitting and/or material-fitting manner.

7. The sterilization sieve basket (1) according to claim 6, **characterized in that** the sterilization sieve basket (1) has at two opposite rim/edge portions, a respective internal sieve basket handle (5) and an associated fixing portion and/or press-on portion (6) or element to be movable and/or deformable in the selected movement in opposite directions, respectively, outside of the sterilization sieve basket, in particular to protrude outward in the fixed position relative to the outer side (4) of the sieve basket wall (3) in two opposite directions.

8. The sterilization sieve basket system comprising a sterilization sieve basket lid and a sterilization sieve basket (1) according to one of claims 1 to 7.

9. A container system (100) comprising a container (101, 102) and a sterilization sieve basket (1) according to one of claims 1 to 7, wherein the sterilization sieve basket (1) is insertable into a container tray (101) of the container and is adapted in such a way that the fixing portion and/or press-on portion (6) or the element in the fixed position abuts the inner wall (104) of the container tray (101) and braces the sterilization sieve basket (1) against the container tray (101) and thus positionally fixes and secures the sterilization sieve basket (1) in the container (101, 102).

## Revendications

1. Panier de stérilisation (1), qui est prévu et conçu pour être inséré dans et retiré d'une cuve de conteneur (101), pour lequel au moins une poignée de panier (5), de préférence en forme d'étrier ou d'anse, est montée de manière mobile, en particulier de manière pivotante, au niveau d'au moins une paroi latérale de panier (3), qui présente une section de préhension (7) qui est connectée, par l'intermédiaire d'une barrette de liaison (18) orientée selon un angle par rapport à la section de préhension (7), à une section de palier (11) au niveau de laquelle la poignée de panier (5) est maintenue au niveau d'un palier (10) côté paroi de panier et peut être déplacée, en particulier pivotée, dans au moins une position de portage et dans une position de fixation, et le panier de stérilisation (1) présente au moins une section de fixation et/ou de pression (6) ou un élément espacé de la section de préhension (7), qui est monté(e) au niveau de l'au moins une paroi latérale de panier (3) et qui peut être déplacé(e) et/ou déformé(e) par un déplacement sélectionné de la poignée de panier (5) depuis la paroi latérale de panier (3) en direction de l'extérieur du panier de stérilisation (1) pour reposer contre une cuve de conteneur (101), **caractérisé en ce qu'**un évidement (9) est réalisé dans l'au moins une paroi latérale de panier (3) dans la zone de l'au moins une poignée de panier (5), évidement à travers lequel la section de fixation et/ou de pression (6) ou l'élément peut être déplacé(e) et/ou déformé(e) par le déplacement sélectionné de la poignée de panier (5) en direction de l'extérieur du panier de stérilisation (1) et fait saillie vers l'extérieur, en particulier dans la position de fixation, par rapport à la paroi latérale de panier

2. Panier de stérilisation (1) selon la revendication 1, **caractérisé en ce que** la poignée de panier (5) est conçue à la manière d'un levier à deux côtés et articulée de manière pivotante autour d'une articulation rotative (10) avec un axe de rotation (8) et la poignée de panier (5) présente la section de fixation et/ou de pression (6) ou l'élément espacé(e) de l'axe de rotation (8) et la section de préhension (7), en particulier en forme de barre, espacée de l'axe de rotation, pour saisir manuellement la poignée de panier (5), dans lequel la section de fixation et/ou de pression (6) ou l'élément est disposé(e) d'un côté de l'axe de rotation (8) et la section de préhension de l'autre côté de l'axe de rotation (8), de sorte qu'un mouvement de pivotement de la section de préhension (7) provoque un mouvement de pivotement de même sens de la section de fixation et/ou de pression (6) ou de l'élément autour de l'axe de rotation.

3. Panier de stérilisation (1) selon la revendication 2, **caractérisé en ce que** la poignée de panier (5) est conçue sous la forme d'un levier droit et/ou une distance verticale entre la section de préhension (7) et l'axe de rotation (8) est nettement supérieure, en particulier de 2 à 6 fois, en particulier de 3 à 4 fois, à une distance verticale entre la section de fixation et/ou de pression (6) ou l'élément et l'axe de rotation (8) pour former un bras de levier efficace.

4. Panier de stérilisation (1) selon l'une quelconque des revendications précédentes, **caractérisé en ce que** la poignée de panier (5) présente une pluralité de sections de fixation et/ou de pression (6) ou d'éléments, de préférence deux ou trois, qui présentent une même distance verticale entre la section de fixation et/ou de pression (6) ou l'élément d'une part et l'axe de rotation (8) d'autre part, et qui sont de préférence disposés de manière axisymétrique le long de l'axe de rotation (8).

5. Panier de stérilisation (1) selon l'une quelconque des revendications précédentes, **caractérisé en ce que** la section de fixation et/ou de pression (6) ou l'élément présente un embout avec une élasticité propre qui présente de préférence du caoutchouc, du silicone et/ou du plastique en tant que matériau et qui est notamment constitué de caoutchouc, de silicone ou de plastique, et l'embout peut être couplé de préférence de manière amovible au niveau de la section de fixation (6), en particulier peut être enfiché et/ou clipsé sur la section de fixation (6).

6. Panier de stérilisation (1) selon l'une quelconque des revendications précédentes, **caractérisé en ce que** le panier de stérilisation (1) présente au moins un module de montage de poignée de panier (16) qui présente le palier (10), la poignée de panier (5) montée dans le palier (10) et une structure de montage (17) qui, en tant que module séparé, est fixé à la paroi de panier (3) au moyen de la structure de montage (17) par complémentarité de formes et/ou par complémentarité de matière, en particulier au moyen d'un raccord à vis, d'une liaison par rivet et/ou d'une liaison soudée.

7. Panier de stérilisation (1) selon la revendication 6, **caractérisé en ce que** le panier de stérilisation (1) présente, au niveau de deux sections de bord/arête opposées, respectivement une poignée de panier (5) située à l'intérieur et une section de fixation et/ou de pression (6) ou un élément correspondant pour pouvoir être déplacés et/ou déformés lors du déplacement sélectionné respectivement dans des directions opposées à l'extérieur du panier de stérilisation, en particulier pour faire saillie vers l'extérieur dans deux directions opposées dans la position de fixation par rapport au côté extérieur (4) de la paroi de panier (3).

8. Système de panier de stérilisation avec un couvercle de panier de stérilisation et un panier de stérilisation (1) selon l'une quelconque des revendications 1 à 7.

9. Système de conteneur (100) avec un conteneur (101, 102) et avec un panier de stérilisation (1) selon l'une quelconque des revendications 1 à 7, dans lequel le panier de stérilisation (1) peut être inséré dans une cuve de conteneur (101) du conteneur et est adapté de sorte que, dans la position de fixation, la section de fixation et/ou de pression (6) ou l'élément repose contre la paroi intérieure (104) de la cuve de conteneur (101) et serre le panier de stérilisation (1) contre la cuve de conteneur (101), fixant et sécurisant ainsi la position du panier de stérilisation (1) dans le conteneur (101, 102).
